(19) Europäisches Patentamt
European Patent Office
Office européen des brevets

(11) EP 3 347 061 B1

# (12) EUROPEAN PATENT SPECIFICATION

(45) Date of publication and mention of the grant of the patent:
**19.05.2021 Bulletin 2021/20**

(21) Application number: **16763035.9**

(22) Date of filing: **08.09.2016**

(51) Int Cl.:
***A61L 27/18*** *(2006.01)* ***A61L 27/20*** *(2006.01)*
***A61L 27/22*** *(2006.01)* ***A61L 27/26*** *(2006.01)*
***A61L 27/38*** *(2006.01)* ***A61L 27/52*** *(2006.01)*
***A61L 27/56*** *(2006.01)* ***C08L 5/02*** *(2006.01)*
***C08L 5/06*** *(2006.01)* ***C08L 5/08*** *(2006.01)*
***C08L 71/02*** *(2006.01)*

(86) International application number:
**PCT/EP2016/071238**

(87) International publication number:
**WO 2017/042301 (16.03.2017 Gazette 2017/11)**

## (54) INJECTABLE MACROPOROUS HYDROGELS

INJIZIERBARE MAKROPORÖSE HYDROGELE

HYDROGELS MACROPOREUX INJECTABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2015 EP 15184552**

(43) Date of publication of application:
**18.07.2018 Bulletin 2018/29**

(73) Proprietor: **ETH Zürich**
**8092 Zürich (CH)**

(72) Inventors:
- **BROGUIERE, Nicolas**
**8004 Zurich (CH)**
- **ZENOBI-WONG, Marcy**
**8049 Zurich (CH)**

(74) Representative: **Schulz Junghans Patentanwälte PartGmbB**
**Großbeerenstraße 71**
**10963 Berlin (DE)**

(56) References cited:
**US-A1- 2014 343 324**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to macroporous hydrogels and their use in tissue engineering and regenerative medicine.

## Background of the invention

Macroporous hydrogels are important in the field of tissue engineering because they have important advantages over homogeneous materials in terms of mechanical properties, stability, molecule diffusion/transport properties and cell invasion. Established methods to form macroporous hydrogels (salt leaching, freeze drying, gas pocket formation) nevertheless typically need prior material templating in harsh conditions, followed by cell seeding; and they are not injectable, which induces more trauma during eventual implantation in a patient. The few methods reported to produce cell-compatible injectable hydrogels (e.g. microparticle fusion, or sacrificial porogen inclusion and degradation) have respectively a very heavy preprocessing step, to form gel particles, or a slow pore formation because of slow porogen degradation. They also have limited control over the pore sizes accessible. Two-phase systems have also been used to produce porous gels, but not in conditions that are compatible with injectability and cell encapsulation US2014343324 discloses biomaterials comprising polymeric networks / matrices, which are formed by reacting a first polyethylene glycol having nucleophilic groups with a second polyethylene glycol having electrophilic groups.

The objective of the present invention is to improve on the state of the art and to provide injectable, tissue-compatible macroporous hydrogels for use in regenerative medicine. This objective is attained by the subject matter of the independent claims.

## Terms and definitions

In the context of the present specification, the term *polyalkyloxazoline* (POx) refers to a polymer of an alkyl derivative (particularly a methyl-, ethyl- or propyl-substituted) oxazoline or oxazine.

In the context of the present specification, *polyethylene glycol* (PEG) refers to a polymer of general formula $H-(O-CH_2-CH_2)_n-OH$ (CAS No. 25322-68-3).

In the context of the present specification, the term *star-shaped PEG/POx* refers to branched polymers consisting of several (more than three) linear PEG/POx chains connected to a central core. The core of the polymer can be a single atom, molecule or macromolecule. Particularly common for linking polyethylene glycol or polyalkyloxazoline chains are pentaerythritol (CAS 115-77-5), 2-hydroxymethyl-1,3-propanediol (CAS 4704-94-3), dipentaerythritol (CAS126-58-9), tripentaerythritol (CAS 78-24-0), glycerol, tetrakis(bromomethyl)ethylene (CAS 30432-16-7), 1,3,5 tribromophenyl, 1,3,5 trihydroxyphenyl, silicon, and dialkylsilane moieties.

In the context of the present specification, the term *hyaluronan* (or its synonyms hyaluronic acid or hyaluronate), abbreviated HA, refers to a polymer of a repeat unit comprising a D-glucuronic acid moiety and a N-acetylglucosamine moiety in alternating β-1,3 and β-1,4 linkage of general formula

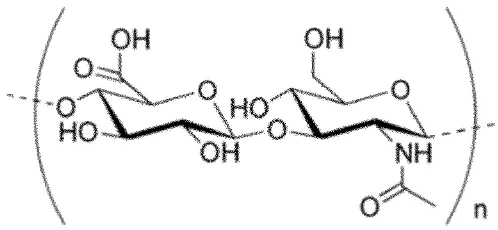

The molecular weight of the repeat unit is 379 g/mol. Hyaluronan in the body can exceed 2500 repeat units (n) per molecule. Hyaluronan is referenced under CAS Nos. 9004-61-9 (acid), 9067-32-7 (sodium salt) and 31700-91-4 (potassium salt).

*Mannuronan* (MA) refers to a linear polymer of β-D-mannuronate residues linked by (1-4) linkages (CAS No. 6814-36-4).

It is understood that when used herein, the term polymer for any of the polymers recited herein, may imply a polymer of the mentioned monomers, some of which are substituted by reactive groups to allow crosslinking.

Molecular mass values (number average molecular weight), where used herein to define polymers or other macromolecules, are determined by gel permeation chromatography (GPC) using nearly monodisperse standards (polydispersity index between 1.0 and 1.1) of the same polymer for calibration, as described in detail in 'Modern Size-Exclusion Liquid Chromatography: Practice of Gel Permeation and Gel Filtration Chromatography' by Andre Striegel, Wallace W. Yau, Joseph J. Kirkland and Donald D. Bly, John Wiley & Sons, 2009, unless stated otherwise. The mass of the standards is measured using matrix-assisted laser desorption/ionization - time of flight mass spectroscopy (MALDI-TOF).

In the context of the present specification, the term *kosmotropic agent* refers to any agent which in aqueous solutions can form diphasic systems with aqueous solutions of PEG or POx.

## Summary of the invention

According to a first aspect of the invention, a method for providing a macroporous gel is provided, comprising the steps of

a) providing an aqueous solution comprising a first and a second solute, wherein the first solute is a cross-linkable polymer selected from a cross-linkable derivative of a polyethylene glycol (PEG) and a cross-linkable derivative of a polyoxazoline (POx);

and the second solute is a polysaccharide kosmotropic agent selected from the group comprising hyaluronan, mannuronan, carrageenan, heparin, chondroitin sulfate, dextran, hydroxypropyl dextran, pectin, alginate, gellan gum, cellulose, methylcellulose, ethylcellulose, cellulose acetate, starch and hydroxypropyl starch, wherein the concentration of said polysaccharide kosmotropic agent in said aqueous solution is 0.1 to 50% (w/v); and

b) adding to this mixture a cross-linking reagent able to cross-link the cross-linkable polymer, thus simultaneously initiating gelation of the cross-linkable polymer and phase-separation.

In certain embodiments, the cross-linkable polymer is selected from the group comprising a star-shaped polyethylene glycol, a polyethylene glycol diblock copolymer and a polyethylene glycol triblock copolymer; or the group comprising a polymethyloxazoline, a polyethyloxazoline, a polypropyloxazoline and a polybutyloxazoline; or the group comprising a star-shaped polyoxazoline and a copolymer of oxazoline, particularly a copolymer of 2-methyl oxazoline and a 2-ethyl oxazoline with a carboxylic acid or alcohol containing oxazoline, particularly 2-carboxyethyl oxazoline or 2-hydroxyethyl oxazoline; or the group comprising partially hydrolyzed poly 2-methyl oxazoline or poly 2-ethyl oxazoline.

In certain embodiments, the cross-linkable polymer is a linear polyethylene glycol.

In certain embodiments, the cross-linkable polymer is a star-shaped polyethylene glycol that has a pentaerythritol core and a molecular weight of 10 to 40 kg/mol, particularly of approx. 20 kg/mol (such as Sigma product number JKA7025). In certain embodiments, the cross-linkable polymer is a star-shaped polyethylene glycol of 10 to 40 kg/mol that has a dipentaerythritol core (such as Sigma product number JKA10034). In certain embodiments, the cross-linkable polymer is a star-shaped polyethylene glycol that has a glycerol core.

In certain embodiments, the cross-linkable polymer is a polyethylene glycol diblock copolymer comprising a copolymer of polyethylene glycol and polyethylene (PE) or polylactide (PLA) or polylactide-co-glycolide (PLGA) or poly-ε-caprolactone (PCL) or polystyrene.

In certain embodiments, the cross-linkable polymer is a polyethylene glycol triblock copolymer comprising a copolymer of polyethylene glycol and polypropylene glycol (PPG) or a copolymer of polyethylene glycol and polylactide-co-glycolide (PLGA).

In certain embodiments, the cross-linkable polymer is a polyethylene glycol copolymer comprising at least 50% PEG and at least one other polymer selected from PCL, PPG and PLGA.

In certain embodiments, the cross-linkable polymer is a polyoxazoline comprising a random copolymer of a methyloxazoline or ethyloxazoline with carboxyethyloxazoline, and the carboxylic acids are substituted with reactive moieties, particularly substituted with one or several reactive moieties selected from the group consisting of a thiol, a vinylsulfone, a maleimide, a transglutaminase substrate peptide, a tyramine, a dopamine, an acrylate, a methacrylate, a vinyl ester, an alkyne, an azide, an aldehyde, and an acrylamide, more particularly from the group consisting of a thiol, a vinylsulfone, a maleimide, an acrylate, and a methacrylate.

In certain embodiments, the cross-linkable polymer is a star-shaped polyoxazoline polymerized from a tetrakis(bromomethyl)ethylene initiator. In certain embodiments, the cross-linkable polymer is a star-shaped polyoxazoline polymerized from a 1,3,5-tribromophenyl initiator.

In certain embodiments, any of the star-shaped polyoxazoline polymers mentioned herein is terminated with a hydroxyl, amino or carboxylic acid group, which in certain further embodiments is further substituted with one or several reactive moieties as specified herein, particularly selected from a thiol, a vinylsulfone, a maleimide, a transglutaminase substrate peptide, a tyramine, a dopamine, an acrylate, a methacrylate, a vinyl ester, an alkyne, an azide, an aldehyde, and an acrylamide. In certain alternative embodiments, the star-shaped polyoxazolines are directly terminated with reactive moieties by quenching the oxazoline polymerization with the desired cross-linkable group.

In certain embodiments, linear polyoxazoline is partially hydrolysed to remove 1 to 40% of its side chains (e.g. acetate release from poly(2-methyl-2-oxazoline)), by boiling in water in the presence of an acid or a base, and the released secondary amines are substituted with reactive moieties.

In certain embodiments, the cross-linkable polymer comprises a reactive moiety selected from the group comprising acrylate, methacrylate, acrylamide, vinyl ester, maleimide, vinyl sulfone, alkyne, azide, aldehyde, tyramine, dopamine and thiol moieties. The reactive moiety is either added at the end of the chains of a star-shaped polymer, or on approximately 5 to 25% of the side chains on a linear polymer.

In certain embodiments, the cross-linkable polymer comprises as a reactive moiety transglutaminase substrate peptides, specifically blood coagulation factor XIII substrate peptides, more specifically peptides containing the sequences FKGG and NQEQVSPL.

In certain embodiments, the cross-linkable polymer is vinylsulfone-modified and the cross-linking reagent is a short linker molecule comprising two thiol (-SH) moieties, particularly a molecule comprising two SH groups separated by an alkyl or heteroalkyl chain comprising two to ten carbon or hetero atoms, or a peptide comprising two cysteine amino acid building blocks.

In certain embodiments, the cross-linkable polymer is vinylsulfone-modified and said cross-linking reagent is selected from the group comprising a hydrophilic polymer substituted with thiol moieties, particularly primary thiol moieties, particularly a star-shaped polyethylene glycol comprising primary thiol ($-CH_2SH$) moieties

or a linear polyethylene glycol comprising primary thiol ($-CH_2SH$) moieties, and a peptide containing two to one hundred, particularly two to twenty, more particularly four to sixteen amino acids, wherein two of said amino acids are cysteines; particularly a peptide amenable to cleavage by endopeptidases, more particularly matrix metalloproteinases, even more particularly a peptide of the amino acid sequence GCRD-GPQGIWGQ-DRCG or GCRE-GPQGIAGQ-ERCG.

In certain embodiments, the molecular weight of the cross-linkable polymer is 1,000 to 1,000,000 g/mol, particularly 10,000 to 40,000 g/mol.

In certain embodiments, the cross-linkable polymer is a linear polymer 50 to 1000 monomers in length, particularly 75 to 500 monomers, even more particularly 100 to 300 monomers, most particularly 150 monomers in length.

In certain embodiments, the concentration of the cross-linkable polymer in the aqueous solution is 0.2% to 50% (w/v), particularly 0.5% to 10% (w/v).

In certain embodiments, the aqueous solution comprises 0.5% to 3% (w/v) of said cross-linkable derivative of a polyethylene glycol or 1% to 10% (w/v) of said cross-linkable derivative of a polyoxazoline.

The kosmotropic agent is a polysaccharide kosmotropic agent selected from the group comprising hyaluronan, mannuronan, carrageenan, heparin, chondroitin sulfate, dextran, hydroxypropyl dextran, pectin, alginate, gellan gum, cellulose, methylcellulose, ethylcellulose, cellulose acetate, starch, hydroxypropyl starch, and wherein particularly said polysaccharide is characterized by a molecular weight in the range from 1,000 g/mol to 10,000,000 g/mol, more particularly 10,000 g/mol to 2,000,000 g/mol, even more particularly 100,000 g/mol to 1,000,000 g/mol, even more particularly approx. 150,000 g/mol.

The concentration of the polysaccharide kosmotropic agent in the aqueous solution is 0.1% to 50% (w/v), particularly 0.1% to 5%, even more particularly 0.35% to 0.5% (w/v).

In certain embodiments, the aqueous solution comprises 0.1% to 1% hyaluronan, particularly approx. 0.4% hyaluronan, or 0.5% to 5% mannuronan, particularly approx. 2% mannuronan, or 0.1% to 10% dextran or a mixture thereof.

In certain embodiments, the crosslinkable polymer is 20.000 g/mol 4-arm-PEG-vinylsulfone, the polysaccharide is mannuronan, and the cross-linking reagent is a matrix metalloproteinase cleavable peptide, particularly a peptide comprising the amino acid sequence GPQGIWGQ. In certain embodiments, the crosslinkable polymer is 20.000 g/mol 4-arm-PEG-vinylsulfone, the polysaccharide is mannuronan, and the cross-linking reagent is the peptide GCRD-GPQGIWGQ-DRCG.

In certain embodiments, the crosslinkable polymer is an acrylate, methacrylate, acrylamide, vinyl ester, or maleimide derivative, and a radical initatior is used to trigger gelation upon heating or exposure to light. Particularly, linear PEG-diacrylate or PEG-dimethacrylate of 1 to 10 kg/mol or POx-diacrylate or POx-dimethacrylate of 1 to 10 kg/mol in the presence of a light triggered radical initiator.

In certain embodiments, the radical initiator is Irgacure 2959 at concentrations of 0.01% to 1% (w/v), particularly 0.025% to 0.1% (w/v), and blue to near UV light is used to trigger crosslinking, particularly light at around 365 nm and 10 $mW/cm^2$.

In certain embodiments, the radical initiator is 2,2-dimethyl-2-phenyl-acetophenone, particularly in the presence of N-vinyl pyrolidone, or lithium acylphosphinate, or rose bengale in the presence of an amine such as triethanolamine in the presence of N-vinyl pyrolidone.

In certain embodiments, the crosslinkable polymer is a transglutaminase substrate peptide derivative, and a transglutaminase is added to trigger gelation. Particularly, star-shaped PEG terminated with peptides containing the sequences FKGG and NQEQVSPL are cross-linked with the addition of the activated transglutaminase blood coagulation factor XIII.

In certain embodiments, the crosslinkable polymer is a tyramine or dopamine derivative, and horseradish peroxidase with hydrogen peroxide is used to trigger the cross-linking. Alternatively, or a photosensitive singlet oxygen initiator such as rose bengale, eosin-Y, methylene blue, or riboflavin is used combined with visible light exposure in the visible or near-UV. Particularly, concentrations of riboflavin of 1-100 $\mu$mol/L and of rose bengale, eosin-Y or methylene blue of 0.01 to 1% (w/v) are used and exposed to near UV/visible light to trigger gelling. Horseradish peroxidase is used at 0.1 to 10 Units/mL with 0.01 to 1 mmol/L hydrogen peroxide, particularly 1 Unit/mL with 0.1 mmol/L hydrogen peroxide.

In certain embodiments, the aqueous solution is characterized by a pH of 7.0-8.0, particularly approx. 7.4; and addition of said cross-linking agent occurs at 20°C to 40°C, particularly approx. 37°C.

In certain embodiments, a cell, particularly a mammalian cell, is added to the aqueous solution prior to cross-linking.

In certain embodiments, the aqueous mixture is passed through a syringe 10 seconds to 15 min after addition of the cross-linking agent and/or a solid macroporous gel is observed 1-60 min after addition of the cross-linking agent.

In certain embodiments, the cross-linking agent and the cross-linkable polymer are loaded separately in a double-barrel syringe, with the kosmotropic agent on either side, and the two barrels are mixed upon injection, resulting in the formation of a solid macroporous gel after 1-30 min.

According to a second aspect of the invention, a macroporous hydrogel is provided, characterized in that it is comprised of a cross-linked polymer, wherein said cross-linked polymer is a derivative of a polyethylene glycol or a derivative of a polyoxazoline; and it exhibits

interconnected macropores characterized by a size of 200 nm to 1000 μm, particularly characterized by a size of 500 nm to 100 μm; and the gel is further characterized by a pH of 7.0 to 8.0, particularly approx. 7.4.

In certain embodiments, the gel is made of any one of the polymers mentioned above as crosslinkable polymers.

In certain embodiments, the macroporous hydrogel is transparent; and its stiffness is 1-500,000 Pa, specifically 10-10,000 Pa, specifically 10-1000 Pa, even more specifically 50-500 Pa.

In certain embodiments, the macroporous hydrogel comprises a viable cell.

In certain embodiments, the macroporous hydrogel comprises a neuron comprising functional, synaptically connected neurites.

In certain embodiments, the macroporous hydrogel is used in a method of treatment of nerve or spinal cord injury, wherein said gel is either grafted or gelled in situ at the site of injury, with or without an additional supporting conduit.

In certain embodiments, the macroporous hydrogel is used to support tissue regeneration. Particularly, the macroporous hydrogel is precultured with or without cells and grafted, or the macroporous hydrogel is gelled in situ at the site of injury with or without cells, to support regeneration. More particularly, neurons, neural stem cells, neural progenitor cells, astrocytes, oligodendrocytes, fibroblasts, endothelial cells, osteocytes, chondrocytes, mesenchymal stem cells, or myoblasts are encapsulated in the gel which is delivered in nerve, spinal cord, skin, bone, cartilage or intervertebral disk.

**Detailed description of the invention**

In this invention, an aqueous solution of two solutes is provided. The two solutes are known to be able to form an aqueous two-phase system. The concentrations of the two solutes are not high enough to generate phase-separation, so they are initially miscible. One of the two solutes is a polymer, which is then chemically cross-linked, which implies that the polymer molecular weight increases over time, resulting in gel formation. But in this process, the increase in molecular weight triggers phase-separation, which in most cases is favored by higher molecular weights. This results in dynamic pore formation during the gelling process. This process is compatible with injection into an animal or patient, and with cell encapsulation. In typical implementations, star shaped polyethylene glycol (PEG) or poly methyl- or ethyl-oxazolines (POx) are modified with vinylsulfones and used as the cross-linkable polymer that will form the gel phase in the presence of a cross-linker (molecule containing two or more thiols). The second solute is a polysaccharide kosmotropic agent which can form aqueous two-phase systems with PEG and POx. The second solute is a polysaccharide such as hyaluronan, mannuronan and dextran. Several PEG/Pox derivatives or several kosmotropic agents can also be combined. These three components are mixed together, initially forming a homogeneous solution. This mixture can be used to suspend cells, and can be injected or put in shape in a mold or on a cell culture support, as it is a homogeneous liquid solution. With the progress of cross-linking, phase separation is triggered, with a cross-linked polymer (PEG/POx) rich phase and a polysaccharide rich phase. Only the PEG/POx phase will result in a gel, the polysaccharide phase will remain liquid, thereby creating a macroporous hydrogel.

In all embodiments, the cross-linkable polymer comprises, has been modified with, a reactive moiety that can be cross-linked.

Covalent crosslinking is achieved through any chemical means known to the skilled artisan, including but not limited to: free radical polymerization of methacrylates, acrylates, acrylamides, vinyl esters, or other ene containing molecules; thiol-ene addition, Michael addition on other acceptors such as maleimides or acrylates. The thiol can be any peptide or protein containing several thiols, typically but not necessarily introduced through cysteine residues. The thiol component can also be a thiolated polymer such as linear or branched PEG with thiol ends, or POx with either thiols on side chains or on endings with linear or branched shape. Enzymatic cross-linking through oxidation of tyramines or dopamine, or with a transglutaminase can also be used. Other classical means would be Schiff base formation from an aldehyde an amino group, azide-yne click copper catalyzed or strain promoted chemistry, Diels alder reaction, 2+2 cycloaddition, amine azide or hydrazide conjugation onto carbodiimide activated carboxylic acids, and others. Cross-linking could also happen through non-covalent interactions such as hydrophobic interactions (e.g. pluronics), or specific recognition (e.g. streptavidin-biotin).

Any other polysaccharide or polysaccharide derivative could be used for the second, kosmotropic solute, such as native/natural polysaccharides exemplified by pectin, alginate, gellan gum, cellulose, mannuronan, hyaluronan, dextran, or derivatives of any of these preceding natural polysaccharides. These polysaccharides are characterized by a molecular weight in the range from 1,000 g/mol to 10,000,000 g/mol, particularly 10,000 g/mol to 2,000,000 g/mol, more particularly 100,000 g/mol to 1,000,000 g/mol, even more particularly approx. 150,000 g/mol. The polymer excluding phase can also be a synthetic new polysaccharide polymer, as long as the phase separation is not lost.

The method of the invention is very cheap easy and fast to use and trivial to scale, which is not the case of other methods. In contrast to the methods and gels of the prior art, the method and gels of the invention simultaneously provide all the desired properties of an injectable macroporous hydrogel. These include biocompatibility with cell encapsulation, in situ pore formation (including in vivo in a patient, in theory), full tunability of the

macropore size over a very wide range and easy handling. Additionally, the method also provides interconnectivity of the pores, perfect hydrogel transparency, outstanding hydrogel permissivity to cell invasion and improved stability.

The straightforward use is in vitro 3D culture for screenings. The hydrogels are performing as well as current gold standards, in particularly for 3D neuron cultures, even though other alternatives exist, such as fibrin gels, collagen gels, and matrigel. These matrices are nevertheless animal derived, which poses serious problems of risk of immunogenicity or virus contamination as well as higher costs, and they are usually too quickly degraded in the presence of cells, all of which is overcome by the present hydrogels. No other fully synthetic hydrogels have been described to provide excellent viability and 3D neurite outgrowth of encapsulated neurons as is the case here. For in vivo delivery of cells and macroporous gel formation, the known alternative methods have serious limitations that only this invention overcomes. No solution previously given was enabling injectable macroporous hydrogels with easy up-scalable handling, direct pore formation, and cell compatibility. The current protocol is also particularly simple to put in place, and more flexible with regards to the range of pore size and stiffness easily accessible.

The invention is further illustrated by the following examples and figures, from which further embodiments and advantages can be drawn. These examples are meant to illustrate the invention but not to limit its scope.

## Examples

A protocol, which demonstrates unprecedented 3D neurite outgrowth and stability in the presence of encapsulated DRGs and dissociated central neurons (primary rat cortical and human iPSC derived) based on the formation of an interconnected macroporous network is provided. The key to this protocol is the use of non-modified high molecular weight polysaccharides (PSs) for polyethylene glycol (PEG) exclusion (hyaluronan (HA) or the non-interactive mannuronan (MA)). Exclusion of the PEG from the PS phase improves the kinetics of the cross-linking by Michael addition of thiols on vinyl sulfones (VS), so that gelation proceeds within minutes in media at physiological pH, instead of requiring pH 8.0 which is damaging to sensitive cells such as neurons.

The phase separation between the PEG and polysaccharides also results in gels with increased stability and neurite outgrowth compared to the non-microstructured equivalents. Adhesion cues such as proteins or integrin binding peptides are unnecessary in this system, exemplifying the overwhelming importance of physical properties in engineering a neurite permissive ECM. In optimized conditions, encapsulated cortical neurons extended neurites at a typical rate of 100 μm/neuron/day since the first week and formed synapses in the synthetic matrix, which resulted in 3D neural networks with spontaneous coordinated electrical activity after two weeks, staying stable for at least a month in culture.

### Microstructuring of PEG hydrogels with PSs

PEG has been long known to form aqueous biphasic systems with dextran and some salts, due to its water organizing properties. This physical effect has been used extensively for protein extractions, and shown to be useful for gel microsphere formation, but its potential for in situ self-assembly of macroporous scaffolds has not been shown yet. This is probably because the phase separation typically proceeds through fast microsphere formation, with subsequent fusion and decantation, which doesn't provide percolating networks of pores and gel. Interconnected networks could be obtained by photocrosslinking overnight under tumbling the dextran of a high percentage PEG-dextran mixture. Despite of its merits, this method does not enable in situ gelling and cell encapsulation, and the resulting scaffolds have low transparency. The inventors found that the key to generate macroporous hydrogels in a way compatible with cell encapsulation and injection was to combine low polymer contents and high viscosities. The low polymer content guarantees that the PEG and polysaccharide solutions are initially miscible. Then, phase separation, which in aqueous systems is highly dependent on the molecular weight, is triggered by the PEG chains elongation happening in the presence of the cross-linker (Fig. 7).

Figure 1 shows two typical implementations of this method, which result in gels with radically different microstructures: 4-arm-PEG-VS (star-shaped polyethylene glycol, vinylsulfone terminated) 1.5% (w/v) cross-linked with MMP-cleavable peptides (containing two cysteines providing thiols for cross-linking by Michael addition) with 70% (v/v) of a manuronan (MA) 2% (w/v) solution or a hyaluronan (HA) 0.5% (w/v) solution respectively (denoted PEG+MA and PEG+HA). The gelling is performed in cell culture medium (Neurobasal+B27) supplemented with HEPES buffer pH7.4. The polysaccharides were chosen for being both chemically inert, available in high molecular weight to guarantee high viscosity at low concentration, and for having no (for MA) to little (for HA) specific biological effect. The concentrations of HA/MA stocks were adjusted to obtain similar complex viscosities over a large frequency range (Fig. 2b), with static viscosities close to 1 Pa (similar to glycerol at RT). The advantage of starting from a homogeneous solution is that the final microstructure does not depend on the exact mixing or injection protocol, as long as the initial solution is well mixed: it is the basis for the formation of reproducible, homogeneous and isotropic pores. To quantify the pore size, connectivity and isotropy, a 4-arm-PEG-VS derivative with a fluorescent tag and confocal laser scanning microscopy (CLSM) was used. PEG gel formed in the absence of polysaccharide appeared perfectly homogeneous (the distance between the PEG

chains is estimated to be 5 to 50 nm and the diffraction limit in the imaging conditions used is 185 nm), whereas the PEG gels formed in the presence of HA or MA were macroporous (Fig. 1a,c). 3D inspection revealed the pores were fully interconnected. The autocorrelation function was computed (Fig. 1e), and found to be isotropic. Pore size quantitation was done by fitting the data with the penetrable sphere model (Fig. If), yielding typical pore sizes of 0.82+/-0.1 μm for PEG+HA and 2.39+/-0.16 μm for PEG+MA. R2 values were >0.8, showing good agreement with the model. The pores volume fraction, S2(0), was found to be 58+/-4% for PEG+MA and 43+/-1.6% for PEG+HA (this last value should be taken with caution as the pores created in the presence of HA are close to the diffraction limit, and therefore appear blurry; the pores in PEG+MA on the other hand are well resolved). It is noteworthy that HA and MA solutions of same viscosity give rise to dramatically different porous structures: this is expected as phase separation kinetics are known to depend not only on viscosity but also on interfacial tension. Pore size could also be adjusted by addition of variable amounts of dextran, which gives faster PEG exclusion without noticeably changing the viscosity, to yield pores of tunable size (data not shown).

The low polymer contents also guarantee small differences of density and optical index between both phases. The former is interesting to avoid settling, which would result in differences between the pore size at the top and bottom of the gels. The latter avoids light diffraction within the gels, which results in perfect gel transparency (Fig. 8), something relatively exceptional for macroporous scaffolds. This enables optical imaging over several millimeters of depth, and easy observation of cells in differential interference contrast (DIC). Such percolating macroporous gels are of strong interest for tissue engineering applications as they have free passageways maximizing transport properties and enabling fast neurite extension or cell invasion, depending on the pore size. Such large interconnected pores are typically found in natural fibrillar matrices like collagen and fibrin, but not in classical chemically cross-linked hydrogels, that typically have 5-50 nm pore size.

Gelling kinetics

An increase in viscosity gives slower diffusion and therefore kinetics. But this effect is more than counterbalanced by the concentration through volume exclusion, and as a result porous PEG gels were formed with enhanced kinetics. It is particularly interesting as this enabled the formation of gels with optimal kinetics at physiological pH, whereas similarly good gelling times are normally only achieved at pH 8.0 (Fig. 2a/c), which is stressful for cells in general and can result in damage or death for sensitive cells such as neurons. The high viscosity also prevents cell sedimentation before gelation occurs.

Dorsal root ganglia (DRG) encapsulation

DRGs are the most common in vitro model of peripheral axon regeneration and, as full explants, are easy to culture due to their resistance to chemical and biological stress. They are commonly grown in 2D to test the bioactivity of various substrates and in 3D in fibrin or collagen, though these tend to degrade within a few days due to the high concentration of proteases around the explant. The investigators found that non-degradable porous PEG gels were permissive to peripheral axon invasion, and stable for extended amounts of time (Fig. 3). For this, DRG explants from E9.5 chick embryo were encapsulated in disks (4 mm x 1.5 mm) of PEG gel formed from 4arm-PEG-VS 0.8% (w/v), cross-linked with stoichiometric amount of PEG dithiol in the presence of HA 0.525% (w/v) and dextran 1.4% (w/v)). Whole gel imaging of the live cell processes (stained by calcein AM) after 30 days revealed that the entire gel had been invaded by a dense mesh of axons, showing permissiveness as well as long-term stability. Cell death was obvious on the surface of the explant but not inside, as seen from the propidium iodide (PI, dead) / Calcein AM (live) staining pattern. This suggests that the outer cell layer helps to protect the inner cells from the stress associated with encapsulation.

3D neural networks from rat cortical neurons in porous PEG

Primary rat cortical neurons were encapsulated in macroporous gels formed with increasing amounts of HA (Fig. 4). The neuron viability, rate of neurite outgrowth, and gel degradation were quantified, as they are the most critical parameters to optimize for 3D neural network formation. The commercial product Q-gel was used as a control of non-porous classical PEG gel, as it represents the current gold standard and has been successful for the encapsulation of many different cell types.

For this series of experiments, since neurons are known to grow best in very soft fibrin and collagen matrices, the investigators chose a concentration of 1.5% (w/v) PEG, which has a storage modulus of 70 Pa just after gelling. They also used MMP-cleavable peptides for cross-linking, as pilot experiments showed markedly reduced viability when using a non-cleavable cross-linker (Fig. 13). In attempts to explain this, the investigators found that TMR-tagged 4-arm-PEG-VS could be taken up by the neurons over the time of gel formation (Fig. 12), and therefore hypothesized that neurons should be able to metabolize the gel fragments to prevent toxicity. This constraint does not apply to neurons which are grown as clusters, since outer neurons provide shielding from PEG exposure, and clusters can be grown directly in non-degradable porous PEG gels (e.g. Fig. 3). The quantifications two days after encapsulation (D2) showed that neurons retained very high viability (>95%) in the porous gels (Fig. 4d), which is an important starting

point, far from reach for what might be the current most successful synthetic neuron supporting gel, Puramatrix. Viability in the Q-gel control was reduced to 70%, probably due to the short exposure to a higher pH as well as to the higher PEG content used in the classical approach. Neurite extension was ~100 μm/neuron on average on D2, independent of the HA percentage (Fig. 4c), which shows the low stiffness chosen is appropriate to support fast neurite extension. Microstructuring had a major influence on the stability of the gels: increasing amounts of HA resulted in less macroscopic degradation (Fig. 4b). Gels formed with no HA were fully degraded within two days (which is why this condition is not in Fig. 4), whereas gels formed with 0.525% (w/v) of HA were fully stable after more than a month. This is expected as the microstructured gels have more dense and robust pillars of concentrated PEG gel, separated by weak channels left by uncross-linked HA. This makes these microstructured gels more resistant to degradation, while maintaining their potential to support fast neurite outgrowth.

Hydrogels microstructured with MA had a large, open pore structure that resulted in misshapen cell morphology, as the cell bodies adapt their shape to the multimicrometer sized pores in which they lie and neurites make sharp turns to follow them (Figs. 11, 13). They were therefore not studied further for this application. The submicrometer pores created by HA form instead a thin and dense network in which the cell bodies and neurites appear smooth, as they do in vivo. Although it has long been assumed that laminin or laminin peptides are necessary for neurite outgrowth in 3D hydrogels, the investigators demonstrate here excellent neurite outgrowth in PEG+HA microstructured gels in the absence of any adhesion cues. This is particularly noteworthy given PEG's stealth, anti-adhesive and non-interactive properties. Several lines of data suggest that neurite extension mainly relies on physical properties.

To test whether gels had intrinsic adhesion cues through the presence of HA, it was digested with 1 mg/ml hyaluronidase (HAse) immediately after gel formation. HA is degraded within seconds in these conditions, as seen by rheometry (Fig. 10), and protein diffusion through the gels is not a concern (e.g. full gel immunostaining is easily performed). Furthermore, when MA was selected for phase exclusion microstructuring, neurons still grew although they lack receptors to this marine/bacterial polymer. Additionally, when PEG+HA and PEG+MA gels were covalently functionalized with IKVAV, even at high concentration, the neurite outgrowth was qualitatively similar to controls without IKVAV, whereas on 2D tissue culture plastic controls, the peptides potently induced neurite outgrowth in a concentration dependent manner (Fig. 11). This shows specific cues are not essential on an already optimal physical background. It is also unlikely that neurons adhere non-specifically to proteins entrapped in the gel, as the investigators work in serum free conditions and without growth factors. Initial neurite outgrowth also occurs too fast for matrix deposition by the cells to play a major role (typically, many ~10 μm neurites are visible one hour after encapsulation). Finally, the investigators have ruled out non-selective adhesion to MMP-cleavable peptides: microstructured non-degradable PEG gels which are cross-linked with PEG-dithiol are completely free of amino acids or charges, and still as permissive to the neurite outgrowth, albeit with reduced viability (Fig. 13). Overall, the investigators' data therefore indicates that physical properties rather than specific adhesion cues are of overwhelming importance to enable fast neurite extension in 3D. Variations in macromer content from 1.2 to 1.9% (w/v) (18-250 Pa) did not significantly affect the neurite outgrowth over two or five days (Fig. 14). The softest gels are more unstable, which can result in more damage and neurite loss. Non-porous controls at 1.5 and 1.9% (w/v), gelled at pH 8.0 without HA, were degraded after a few days. Q-gels were perfectly stable, but did not enable nearly as much neurite outgrowth as porous PEGs did. In the optimized conditions, encapsulated primary neurons were forming extensive networks within a few days, that increased in density and stayed stable for at least a month (Fig. 5a). The main differentiated neuron markers were expressed similarly to what is known of 2D cultures (Fig. 5b), and a large number of presynaptic densities were present. Solid spiking activity was observed after 16 days of culture using a fluorescent reporter of intracellular calcium. Coordinated activity was clear (Fig. 5c), which proves the formation of 3D networks with functional synaptic connectivity in the synthetic ECM.

<u>Translation to hiPSC derived neurons</u>

Primary rat neuron cultures have long been a reference model for cellular biology studies, but hiPSC derived neurons are emerging as a more relevant model of human neurological conditions. They provide a reproducible source of defined cells in which particular mutations or drug effects can be studied, and they are thought to have therapeutic potential. They are typically grown in defined serum-free media in 2D, but matrigel is generally used for 3D cultures. Here, the investigators establish an effective defined 3D culture system for hiPSC-derived neurons which does not rely on immunogenic materials that would prevent translation to clinical applications. hiPSC derived neurons were encapsulated in PEG gels microstructured with 0.525% (w/v) HA with high viability (up to 85%) and extended neurites at the same high rate as primary neurons (Fig. 6). They were very sensitive to stiffness and cell density, and degraded the gels noticeably faster. As a result, gels were stable for the five days needed to form connected networks, but unfortunately not for the 2-3 weeks necessary to obtain spiking activity. The best compromise, giving a gel stable for more than a week with fast neurite extension and high viability, was of 2% (w/v) PEG and 5 M cell/ml in these microstructuring conditions. Cells appeared fully differentiated, being positive to the neuronal markers neurofilament, βIII-tubulin

and MAP2, and negative to the astrocytic marker GFAP.

DISCUSSION

Many methods have been developed to create scaffolds with macroscopic or hierarchical porosity, typically by salt leaching, freeze-drying or electrospinning. However these methods do not enable cell encapsulation or in situ formation. Here, the investigators present a unique method which allows direct encapsulation of cells into porous structures which are created by phase separation between PEG and high viscous polysaccharides as the PEG undergoes step growth polymerization. Defined 3D cultures enabling fast neurite outgrowth and synapse formation are of prime interest for studies relying on neuron-matrix interactions such as axonal guidance, growth cone machinery, synapse assembly and regeneration. The possibility to study the bioactivity of extracellular cues on a blank background is an important tool, especially combined with hiPSC-derived neurons, as those are commercially available from a variety of patient specific and transgenic cell lines that provide important reproducible models of neurological disease. Due to their perfect transparency, the described microstructured gels are especially suited for imaging studies.

Electrically active 3D models also represent a substantial improvement over 2D models. In vitro neural networks are an important tool for the study of electrophysiology, neuromodulation, neurotoxicology or for drug screenings. Their 3D counterparts have dramatically different connectivity, which yields data, which is more relevant to the in vivo situation. Network connectivity was recently studied on stacked 2D surfaces, however, the system described by the investigators goes one step further, by placing the neurons in a fully 3D environment.

The macroporous hydrogels described in this study provided an excellent mimic of the biophysical properties of the neural extracellular matrix, allowing for the first time formation of stable and functional networks in a synthetic PEG gel. For in vivo applications, it is of interest that the components and crosslinking chemistry used here are all biocompatible. In particular, PEG, HA and alginate are already used in the clinics. Due to the straightforward tunability of the system towards the micron range, one can also envision encapsulation of other cell types to control their migration, assembly and interactions in 3D space. The meso-/macroporosity of these hydrogels gives very high surface area, improved mass transport and should allow for the development of more organized tissue analogues compared to homogeneous systems.

METHODS

Unless otherwise stated, all chemicals were from Sigma-Aldrich, cell culture reagents from Invitrogen, experiments were performed in triplicate, and images were minimally modified (brightness/contrast for all pictures, and gamma for neuron pictures only, adjusted in order to make both neurites and cell bodies clearly distinguishable).

4arm-PEG-vinylsulfone (4arm-PEG-VS)

1 g (50 μmol) of 20 kDa 4-arm-PEG-thiol (Laysan Bio) was dissolved into 4 ml of triethanolamine (TEOA) buffer, 300 mM, pH8.0, and immediately added dropwise over vigorous stirring to 1004 μl (10 mmol) of divinyl sulfone in 4 ml of the same buffer. The reaction was essentially complete within minutes, and was left to proceed for 2h. The product was dialyzed against mQ-water (at least 6 water changes over at least 6 h each), and lyophilized. Thiols were found to be completely substituted within the detection limit of proton NMR (in deuterated chloroform on a Bruker 400 Mhz instrument). The product was resuspended at 4% (w/v) in mQ water, sterile filtered, aliquoted by 200 μl (8 mg), lyophilized, and stored at -20°C until use.

Matrix metalloproteinase (MMP) cleavable peptide handling

MMP-cleavable peptide (Nagase, H., Biopolymers 40, 339-416, 1996; Lutolf, B. M. P., Adv. Mater. 15, 888-892, 2003) with the sequence Ac-GCRD-GPQGIWGQ-DRCG-NH2 (1746 Da) was from Anawa (Switzerland) in >95% purity. The HPLC purified peptide comes as an acidic TFA salt which needs to be neutralized. For this purpose, ~100 mg of peptide powder was dispersed in a small amount of water (~1 ml), supplemented with a minimal amount of phenol red as a pH indicator, and neutralized (yellow solution turning orange) with 300 mM aqueous NaOH. The protonated form of the peptide is poorly soluble, but the initial slurry becomes clear during processing. It is important to avoid adding an excess of NaOH as thiols oxidize quickly at pH>7.0 (indicator turning reddish orange). The neutralized peptide was then diluted in mQ water to 1% (w/v), sterile filtered, aliquoted by 400 μl (4 mg), lyophilized, sealed and stored at -20°C until use. It was found to be stable for at least 6 months in this form. The peptide powder typically contains a significant amount of counter ions and a few oxidized peptides, so for each batch, the exact concentration of free thiols in a resuspended aliquot was determined with Ellman's assay (Ellman, G. L., Arch. Biochem. Biophys. 70-77, 1959) using a beta-mercaptoethanol calibration curve. The small amount of phenol red used for pH balancing was controlled to have an absorbance negligible in the context of this assay.

Buffers

HEPES buffer (200 mM of HEPES and 100 mM of NaOH in mQ water, adjusted to pH7.4) was prepared with special care as it was used for the gel formation by Michael addition, which is very pH sensitive (Lutolf, M.P.,

Biomacromolecules 4, 713-722, 2003). Absorbance measurements of the pH indicator phenol red at 558/433 nm were found to provide the most reliable pH values, to guarantee reproducible experiments without relying on calibration solutions and electrodes, more prone to drifting. The ratio of absorbance at 558 / 433 nm was adjusted to 2, which corresponds to pH 7.40 at 24°C and 300 mM ionic strength (Yao, W., Environ. Sci. Technol. 35, 1197-201, 2001) (assuming zwitterions are carrying two charges. If instead they are treated like a molecule with no charge, this corresponds to 100 mM ionic strength and pH 7.5). TEOA pH8.0 was prepared similarly, while PBS pH6.0 was prepared by lowering the pH of PBS pH7.4 (Gibco) with HCl 300 mM.

Mannuronan (MA)

High viscous (see figure S1) bacterial MA (Ertesvag, H., Methods Biotechnol. 10, 71-78, 1999) was a kind gift from Prof. Skjåk-Bræk (Norwegian University of Science and Technology, Biopolymer Laboratory). Unlike HA, alginate is not present in mammals, and is therefore not expected to be specifically recognized by any neuron receptor. In addition, this form of alginate is purely viscous, as it is lacking guluronates (that enable calcium cross-linking of wild-type alginates).

Gel formation

Supports were prepared in advance. A 1 mm layer of PDMS (Sylgard 184, 10% (w/w) cross-linker) was heated in a petri dish for 2 h at 50°C. Ring shaped molds were obtained with successive concentric punching at 4 and 6 mm (dermal punches from Kai Medical). 24 well plates were prepared with one sterile coverslip per well, upon which one PDMS mold was placed. The PDMS sticks to the glass and gels can be made directly in this support, which enables easy transfer, imaging, or immunostaining, all without mechanically disturbing the neurons. Attempts to keep the gels free floating as is usual for other cell types gave much poorer results: neurites typically get mechanically broken during media changes or gel transfers. PDMS molds could be recycled after washing with detergent and UV sterilization. Ethanol should be avoided as it can be loaded in the PDMS and released into the culture. Just prior to gel making, aliquots of 4arm-PEG-VS were resuspended at 20% (w/v) in HEPES pH7.4 (giving 40 mM of vinyl sulfones). MMP-cleavable peptides were resuspended at 8% (w/v) in PBS pH6.0 (giving typically 50 mM of thiols according to Ellman's test). The slightly acidic pH of the peptide solution is important to avoid thiol oxidation, which becomes significant in a matter of hours at pH7.4 Aliquots of sterile filtered complete medium supplemented with 0.75% (w/v) hyaluronan (HA, Sigma 53747) were kept at 4°C. A positive displacement pipette such as Gilson Microman M50E is necessary to pipette solutions containing more than 0.5% (w/v) HA accurately due to the high viscosity. To prepare 100 μl of typical gel containing 2% (w/v) PEG, 0.525% (w/v) HA, and 1e7 cells/ml, the stock solutions were combined as follows: 10 μl of 4arm-PEG-VS stock, 12 μl of HEPES pH7.4 buffer, 70 μl of cell suspension at 1.43e7 cells/ml in HA supplemented medium, and finally 8μl of MMP-cleavable peptide stock as a droplet on the side of the tube. After quick vortexing and vigorous mixing with a positive displacement pipette for ~10 seconds, the liquid precursor could be distributed with 15 μl/PDMS mold. Gelation occurs within 3-4 minutes at this concentration, but can take up to 15 min for softer gels, so every gelation was left to proceed for 1h in the cell culture incubator (37°C, 5% CO2) before adding 1 ml growth medium. The gelling mix has physiological pH and osmotic pressure as well as a large proportion of complete medium: as a result, cells are minimally stressed, and neurite extension is already visible in phase contrast when stopping the gelling at 1h. The pH is kept stable by the ~20% (v/v) of strongly buffered HEPES pH7.4 solution. Other conditions are obtained by changing the volumes of 4arm-PEG-VS and MMP peptide stocks added in the mix correspondingly, replacing the buffer by TEOA pH 8.0, changing the HA and cell concentrations in the stock, changing the medium to the one adapted to each cell type, or replacing the HA by another high viscous supplement, as indicated for each experiment. Non-degradable gels were formed by replacing the MMP-cleavable peptides by PEG-dithiol (Laysan Bio, 3.4 kDa) in same buffer and thiol molarity. Laminin α1 (2110 - 2127) peptides with the sequence CSRARKQAASIKVAVSADR-NH2 (designated simply as IKVAV, ANAWA) were added in the indicated cases. To do so, the IKVAV peptide was first added to the 4arm-PEG-VS solution from a 4 mg/ml stock and left to conjugate for 30 min, before the rest of the protocol was performed (in this case, the amount of HEPES buffer added was reduced accordingly to keep the same total volume). A FRAP test with a coumarin-tagged IKVAV peptide was used to verify that the peptide gets successfully anchored in the gel. IKVAV bioactivity was assayed in 2D according to published protocols (Tashiro, K., J. Biol. Chem. 264, 16174-82, 1989).

Dynamic shear measurements were performed on an Anton Paar MCR 301 rheometer. The liquid precursor of the gel was prepared as described above, with complete growth medium but no cells, and inserted between a metal floor plate and a 20 mm diameter parallel plate geometry. The storage G' and loss G'' moduli increase was monitored with 1 Hz oscillation at 5% strain, 0.25 mm gap, 37 °C peltier-controlled temperature and with water-saturated atmosphere. This strain was chosen to fall well within the linear viscoelastic region of the gels (extending at least from 1 to 400% strain at 1 Hz). The stiffness was defined as the storage modulus at 60 min (point at which all samples reached a plateau and at which gelling was stopped by addition of cell culture media), and the gelling time was defined as the time at which half of the plateau stiffness is reached on the log scale,

as indicated in supplementary figure 1 on a typical experimental curve. Complex viscosities of 0.5% (w/v) HA and 2% (w/v) MA in growth medium were measured at 2% strain, 0.2 mm gap, 37 °C and with water saturated atmosphere, between 0.01 and 10 Hz. Tetramethyl rhodamine tagged 4arm-PEG-vinylsulfone (TMR-tagged 4arm-PEG-VS) 100 mg (5 μmol) of 20 kDa 4-arm-PEG-thiol was dissolved in 2 ml of mQ water. 0.12 mg (0.25 μmol) of tetramethyl rhodamine-5-maleimide (sigma 94506) in 2 ml PBS pH7.4 was then added dropwise over stirring. The conjugation happens within seconds, but was left to proceed for 10 additional minutes. The resulting mixture was added dropwise into 220 μl 2.2 mmol) of divinyl sulfone in 4 ml of TEOA buffer 300 mM pH8.0, left to react for 30 min, dialyzed, and lyophilized. All the handling was performed in the dark.

This protocol substitutes 1/80$^{th}$ of the 4-arm-PEG ends in the upper limit case of 100% conjugation efficiency, which insures negligible impact on gel formation.

Porosity imaging and quantification

Gels were made as described above with PEG 1.9% (w/v), no cells, and using TMR-tagged 4arm-PEG-VS. To produce different pore sizes, the medium (70% of the mix) was supplemented with 0 or 0.5% (w/v) of HA or 2% MA. The microstructure of each gel was imaged after 3 days of incubation in PBS pH7.4 on a Leica SP8 confocal microscope with a 63x/1.4 Oil objective and 520 nm excitation, as a cubic stack of 25 μm with 50x50x200 $nm^3$ pixel size. Leica HyDs were used for detection, with the advantage of working in photon counting with very low noise, and therefore of providing a 'true' black level. 3D view of the pore connectivity was obtained with bleach correction, 2 px Gaussian smoothing, thresholding, surface triangulation with the Matlab isosurface and reduce patch functions, and Blender rendering. For pore size quantification, each one of three stacks per condition was filtered with bleach correction (Fiji, simple ratio, background level 0) and 1 px Gaussian smoothing. The stacks are then thresholded and inverted to obtain binary images with pores marked with 1 and fluorescent PEG with 0, and the second order correlation functions $S_2(x,y)$ were calculated with a custom Matlab script. Since isotropy was observed, the data was reduced to $S_2(x,y) = S_2\left(r = \sqrt{x^2+y^2}\right)$, using 50 nm bins for r. Finally, the resulting data was fitted using the penetrable sphere model (Berryman, J. G., J. Appl. Phys. 57, 2374-2384, 1985).

Rat E17 dissociated cortical neurons

Cortices from E17 Wistar rat embryos were dissected and dissociated as described previously (Buerli, T., Nat. Protoc. 2, 3090-101, 2007). Briefly, the minced cortices were incubated for 15 min at 37°C in PBS supplemented with 1 mg/ml BSA, 10 mM glucose, 0.5 μg/ml DNAse (Sigma, D-5025) and 0.5 mg/ml papain (Sigma, P-4762), and washed with blocking medium consisting of DMEM + 10% FBS. They were then resuspended in 2 ml of blocking medium, and dissociated by trituration using a fire polished Pasteur pipette. The dissociated cells were resuspended in serum free growth medium, consisting of Neurobasal + B27 (Gibco 21103-049 and 17504-044) supplemented with 1x Glutamax and Penicillin/Streptomycin, and plated overnight on PLL coated flasks (50 ug/ml PLL in borate buffer, pH8.4, incubated at 37°C for 1h). The following day, the plated neurons were first washed with TrypLE express for 5 min, to detach weakly adhered non-viable cells and cell debris. The remaining healthy cells were then detached with trypsin/EDTA (Gibco 12605-010 and 25200-072). After addition of two volumes of blocking medium, the cells were centrifuged and resuspended in serum-free growth medium. All the subsequent cell culture in 2D or 3D was done in the serum free growth medium, half of it changed once a week.

Dorsal root ganglia (DRG) isolation and culture

DRGs were isolated from E10 chick embryos immediately before encapsulation. They were cultured in the serum free growth medium supplemented with 1mg/ml albumin and 50 ng/ml NGF. Encapsulation was done in 4arm-PEG-VS 0.8% (w/v), cross-linked with stoichiometric amount of PEG-dithiol in the presence of HA 0.525% (w/v) and dextran 1.4% (w/v)) as described in the gel formation section.

Human induced pluripotent stem cell (hiPSC) derived neurons

hiPSC derived neural progenitor cells (Axol ax0016) were propagated and differentiated according to the online supplier protocol. The derived human cerebral cortical neurons were then encapsulated into gels with the same protocol as primary neurons, in the presence of HA 0.525% (w/v) for microstructuring and replacing growth medium by Axol maintenance medium (also serum free).

Spiking imaging with calcium sensor

The medium of the gels for calcium imaging was removed and replaced with fresh complete medium supplemented with 6 μM of Oregon Green BAPTA-1 (Life technologies) from a 1mM solution in dry DMSO. After 1h incubation, the gels were washed in their original medium for 1h, and imaged on a Leica SP8 confocal microscope using a 20x/0.95 water objective with an 8 Khz resonant scanner and irradiation at 488 nm. Resulting imaging rate was 7.44 Hz.

Viability assays

Viability assays were done by supplementing the medium of the gels with calcein AM (green cytoplasm, live cells, 2 μM from 4 mM stock solution in DMSO) and a nuclear stain among Hoechst (blue nuclei, all cells, 10 μg/ml from a 10mg/ml solution in mQ water), ethidium homodimer-1 (red nuclei, dead cells, 2μM from 2 mM stock solution in DMSO/water 1:4), and propidium iodide (red nuclei, dead cells, 6.6 μg/ml from 0.33mg/ml solution in mQ water). The gels were incubated in the supplemented medium for 1h, then washed for 1h in fresh medium and imaged on a Leica SP8 multiphoton microscope with a 20x/0.95 water objective over 200 μM depth for dissociated cells, or with a 10x/0.3 air objective as a single slice for DRG explants. Live and dead cells were counted manually.

Gel degradation

Gel stability was quantified after two days (conditions that would dissolve within a month of culture were already visibly degraded at this point) on 4 mm diameter gel layers, by taking a tile scan covering the whole gel on a Zeiss Observer 2 microscope in bright field with a 5x air objective. Tiles were assembled with Microsoft Image Composite Editor. Degraded areas were clearly visible as they are free of suspended cell bodies and are outlined with dark cell agglomerates that clustered on the sides and bottom of the degraded parts. They were segmented manually using Fiji, and the percentage of degradation is reported as the degraded area over the total gel area (average and standard deviation from 6 samples).

PEG induced clustering and PEG uptake

Cell clustering was observed immediately after mixing when performing neuron encapsulation with 10% (w/v) high molecular weight PEG (Sigma, 200kDa) as a viscous component. Imaging was done with PlasDIC on a 20x Air objective. To study PEG uptake in best visibility conditions, neurons were grown in 2D for 6 days on PLL coated tissue culture plastic, to ensure full recovery from tissue dissociation. Their medium was then supplemented with 1% (w/v) TMR-tagged 4arm-PEG-VS, to mimic the exposure to PEG during encapsulation. The culture was carefully washed 5 times for 10 min with new growth medium, and imaged immediately in PlasDIC and fluorescence on a 20x Air objective to see PEG association with the cells. The same observation was done after 12 additional days in culture, to observe PEG retention. The fluorescent channel was corrected for light inhomogeneity and noise with background substraction and 3 px median filtering.

Neurite outgrowth quantification

Neurite length was quantified with manual tracing at D2 as it gives maximal accuracy and reliability, but with automated tracing at D5 because the length at this time point becomes nearly intractable to manual tracing. Every image from automated tracing was visually checked for appropriate tracing. Manual tracing was done with simple neurite tracer (Longair, M. H., Bioinformatics 27, 2453-2454, 2011) and automated tracing with NeuriteQuant (Dehmelt, L., BMC Neurosci. 12, 100, 2011). Tracing was done on 200 μm deep and 620 μm wide two-photon imaged 3D stacks. Fluorescent staining was βIII-tubulin by default (staining all neurites on fixed samples) and calcein AM (staining all processes on live samples) for series that contained partially degraded gels, that are too weak to withstand immunostaining (HA response curve in Fig. 4 and Fig. 6). The DAPI stained nuclei were counted and used for normalization to cell number.

Immunocytochemistry and imaging

Primary antibodies were: βIII-tubulin (Sigma T5076, 1:500), Neurofilament (Sigma N4142, 1:150), Synaptotagmin (DSHB mAB 30, developed by Dr. Reichardt from UCSF, 8 μg/ml), MAP-2 (Sigma M3696, 1:200), GFAP (R&D Systems AF2594, 10 μg/ml). Secondary antibodies conjugated with Alexa 488 and 594 (Invitrogen A11005, A11008, A10680, A11015) were used at 1:200. The investigators adapted standard immunocytochemistry protocols to stain and image directly the entire gels. Samples were fixed and permeabilized for 1h at 4°C in 10% formalin with 0.1% Triton X-100, blocked overnight with 5% BSA in PBS, and washed with PBS (twice 1h, once overnight). They were then incubated in primary antibody (overnight at RT with gentle shaking, dilution with 3% BSA in PBS), washed with PBS, incubated overnight in secondary antibody, washed with PBS, stained with DAPI 0.3 μM in PBS for 1h, and finally washed with PBS. Imaging of immunostained samples was performed on a Leica SP8 multiphoton microscope with a 20x/0.95 water objective, over 620 μm width and 200 μm depth, using 1 μm step, 600 Hz scan-rate, 1024x1024 px, and simultaneous excitation at 710 nm and 1100 nm using MaiTai Deepsee and Insight Deepsee fs-lasers respectively (Spectra Physics). The two to three colors were collected simultaneously. The resulting stacks were applied a maximum intensity projection (MIP), compensated for light inhomogeneity by dividing by a fluorescein reference image, and had their color display adjusted (brightness/contrast/gamma).

Statistical analysis

One-way ANOVA and Tukey's post hoc testing were used to quantify differences within series, with $p<0.01$ considered significant.

**Description of the Figures**

Figure 1. High viscous polysaccharides form two phase systems with PEG that result in porous gels. (a) CLSM imaging of control gelled without polysaccharide. The structure is perfectly homogeneous and free of pores at a resolution of 185 nm. (b) PEG 1.5% formed in the presence of high viscous HA 0.35% (w/v) has porous structures of 0.5 to 1.5 μm. (c) PEG 1.5% formed in the presence of high viscous MA 1.4% (w/v) has porous structures of several microns. (d) 3D examination of the porous network shows the pores are fully interconnected. (e) Autocorrelation function of the pores. The circular symmetry of the correlation function shows the porous network is isotropic. (f) The autocorrelation function can therefore be analyzed as a function of radius only. The experimental data (dots) for both types of porous gels and their fitting with the penetrable sphere model (lines) are shown. Best fit is obtained for a pore diameter of 0.82 +/- 0.1 μm for HA and 2.39 +/- 0.16 μm for MA. Error bars: standard deviation (n=3). (a-c) are single slices from confocal microscopy. (d) is a rendering from a 25 μm cube in the gel. Scale bars: 5 μm.

Figure 2. Volume exclusion enhances the kinetics of PEG gelling with Michael addition. (a) Rheologic monitoring of gelation. HA supplementation enable gels to form at physiological pH with nearly the same kinetics as obtained at pH8.0 with pure PEG gels. (b) Complex viscosity of stock solutions of HA and MA in complete growth medium at 37°C. (c) Gelation time. Higher PEG content or pH result in faster gelling, as does replacing the gelation media by the viscous HA stock solution. Q-gel is given as a reference of a commercially-available PEG gel. (d) Stiffness after 60 min of gel formation (initial plateau). PEG concentrations between 1.2 and 1.9% (w/v) span a range of stiffness relevant to the developing nervous system, assumed to give best conditions for axonal growth. Q-gel stiffness is also shown after 10 min gelling, as it is the time at which the manufacturer recommends adding media. Error bars: standard deviation (n=3).

Figure 3. Dorsal root ganglia (DRGs) in non-degradable macroporous PEG gels. (a) Whole gel imaging after 30 days of culture shows complete invasion by axons from the DRG (cluster in the center, fluorescence imaging 1,5 mm maximum intensity projection (MIP)). (b) Close-up of the center showing high neurite density (top view, 1.5 mm MIP). (c) Side view of previous showing neurites are distributed through the whole depth of the gel (1.5 mm MIP). The DRG position is highlighted, as only its lower surface is visible, the rest of it and the neurites in a cone behind are less accessible to the light and appear dark. (d) Live/dead imaging of a cross-section of the DRG. A crown of dead cells is visible but the inner cells are alive. Scale bars: 1 mm (a) 250 μm (b) 250 μm (c) 125 μm (d).

Figure 4. Microstructuring with HA improves the stability of soft gels and allows fast neurite outgrowth, gel formation at pH 7.4 and high viability. Hyaluronan can be removed after gelling without killing the neurons, degrading the gel or preventing the neurite outgrowth. (a) Macroscopic gel degradation as seen in brightfield at D2, with degraded parts highlighted in white and PDMS support in green, and close up of the morphology of the neurons as seen by fluorescence microscopy (projections of 200 μm stacks). PEG 1,5% gels formed without HA are not shown because they were not stable for 2 days and therefore could not be quantified. Staining is calcein/DAPI (1 to 3) and βIII-tubulin/ DAPI (4 and 5). Both methods stain all the neurites/cell bodies, but partially degraded gels did not withstand immunostaining, so the cell morphology was directly imaged on calcein stained live samples. (b) Macroscopic degradation at D2 as percentage of the gel area. (c) Total neurite length divided by the number of cell bodies in a sample volume at D2. (d) Viability at D2. Error bars: standard deviation (n=6 for degradation, n=3 for viability and neurite length). *: Different from all other conditions ($p<0.01$). Others are comparable ($p>0.01$). Scale bars: 1 mm (first line) and 25 μm (second line).

Figure 5. Primary cortical neurons from E17 rat embryo encapsulated in porous PEG gels form long-term stable electrically active 3D neural networks. (a) 200 μm MIPs with color-coded depth show that encapsulated neurons are homogeneously distributed and extend neurites in 3D through the matrix. Long neurites are already visible two days after encapsulation, and growth is continuing at increasing rate to form a dense network by D5. By D26, the whole gel is filled with a dense neurite network. (b) Immunocytochemical markers (200 μm MIPs from two-photon laser scanning microscopy stack acquisitions): βIII-tubulin is a neural marker that stains all neurites of embryonic neurons, synaptotagmin a marker of presynaptic terminals, and MAP2 a differentiated neuron marker known to stain only cell bodies and proximal neurites at this time point. (c) Spiking activity in gels is observed with the intracellular calcium fluorescent reporter Oregon Green BAPTA-1. Independent spontaneous activity is observed (e.g. cell bodies 1 to 3) as well as synchronized activity (cell bodies 5 to 11 at 10 s), proving that a functional synaptically connected network was established by 16 days in the synthetic ECM. Scale bars: 100 μm with 200 μm color coded depth (a) and 50 μm (b,c).

Figure 6. Translation of the method to human iPSC derived neurons. (a) Effect of cell density and stiffness in PEG gels structured with 0.525% HA on the cell morphology as seen by two-photon excited fluorescence microscopy (Calcein/Hoechst) at D2 (200 μm MIP). (b-c) Viability and neurite outgrowth are impaired at high stiffness or low cell density. (d) Qualitative description of the gels at D5: softer gels and higher cell densities result in faster degradation, resulting in unstable gels in the most extreme combination. (e) Immunocytochemistry. Virtually all live cells are positive for the neuronal markers neurofilament, βIII-tubulin and MAP2, and negative for the astrocytic marker GFAP. Error bars: standard deviation (n=3). *Statistically different from all other conditions ($p<0.01$). $p>0.01$ otherwise. Scale bars: 40 μm.

Figure 7. Monitoring of the phase separation during gelling. The sample is TMR tagged PEG 1,5% (w/v) gelled at room temperature (slower than the 37°C used in the rest of the paper) in the presence of MA 2% (w/v) using PEG-dithiol as a cross-linker, imaged with CLSM. Time post-mixing is indicated. Scale bar: 5 μm.

Figure 8. Gel transparency as seen in differential interference contrast (DIC). (a) The gels can hardly be distinguished from water immediately after formation (b) The gels are still perfectly transparent except for a few scattered cell debris two weeks and a half after neuron encapsulation. Scale bars: 50 μm.

Figure 9. Typical gelling profile together with the stiffness and gelling time definitions used in this work. The initial stiffness is taken as the stiffness at 60 min, time at which the stiffness approaches a plateau and medium is added to stop the gelling and proceed to cell culture. Gelling time is taken as the time at which the storage modulus has reached half of the maximum on a logarithmic scale - this definition was preferred over the time of cross-over between storage and loss modulus because of the very different viscosities (so loss moduli) of the samples to be compared. The curve is PEG 1.9% gelled in the presence of HA 0.525% (w/v).

Figure 10. Hyaluronan degradation monitoring. Hyaluronan is quickly degraded in medium supplemented with 1 mg/ml HAse, which enables to remove it from porous PEG gels. Error bars: standard deviation (n=3).

Figure 11. Adhesion peptides are not needed when using optimized mechanical conditions in 3D culture. (a) IKVAV peptides promote cell adhesion and neurite extension on tissue culture plastic (TCP) in a dose dependent manner. (b) Neurite outgrowth in porous PEG gels is similar in the absence and in the presence of IKVAV peptide even in high concentration (100 μM shown), irrespective of the polysaccharide used to create the pores. Note the misshapen morphology adopted by neurons in the large pores created by MA. Scale bars: 40 μm.

Figure 12. Interactions of PEG components with neurons (a) Very high molecular weight PEG could not be used as a viscous supplement as it immediately induced clustering of the neurons in suspension. (b) The functionalized high molecular weight star PEG used for gel formation is taken up by neurons, as seen using a TMR-tagged variant. Scale bars: 50 μm.

Figure 13. Neurite outgrowth from encapsulated primary cortical neurons in a porous non-degradable PEG gel. Neurite outgrowth happens even in the absence of MMP cleavable peptide, even though viability is reduced. This also serves as a control that MMP-cleavable peptides were not used for adhesion in the rest of the work, since these gels are only cross-linked with PEG-dithiol in the presence of MA. 200 μm MIP, scale bar: 50 μm.

Figure 14. Neurite outgrowth quantification from βIII-tubulin two-photon imaging. (a) Neurite outgrowth two and five days after encapsulation. No strong stiffness dependence is seen over more than an order of magnitude (20 to 250 Pa) in the presence of HA microstructuring, only softer gels have reduced neurite outgrowth due to partial degradation. Gels made without microstructuring, replacing the accelerating effect of volume exclusion by polysaccharides by a higher pH, degrade within the first days (grey crosses when degraded). Q-gel is used as a reference, both as free floating gels (classical protocol) and as supported gels (identical molds as our protocol). Error bars: SEM (n=3). Fig. 15 shows that the increased stability of macroporous gels is due to stability at a lower cross-linked polymer content rather than a higher resistance to enzymatic degradation. a) Macroporous gels are degraded by the protease at a similar rate as non-macroporous gels (4-arm PEG-vinylsulfone of 20 kg/mol 1.5% (w/v) cross-linked with MMP-sensitive peptides ERCG-GPQGIWGQ-GCRE through Michael addition of thiols on vinylsulfones, in the presence of hyaluronan 1.6 kg/mol of 0.525% (w/v) for the macroporous gel. Digestion solution is papain at 0.5 mg/ml in phosphate buffered saline + 1 mg/ml bovine serum albumin. The reaction is monitored with a texture analyzer used for indentation, and the indentation moduli are normalized to the initial indentation modulus for each measurement). Error bars: standard error from n=3. b) Macroporous gels remain stable at lower cross-linked polymer content than normal gels (4-arm PEG-vinylsulfone of 20 kg/mol at the weight over volume indicated in the legend, cross-linked with PEG-dithiol of 3.8 kg/mol. Macroporous gels formed in the presence of an additional 0.5% (w/v) hyaluronan and 1% (w/v) dextran. The dry mass of the gel is measured after washing salts and unbound polymer with hyaluronidase 1mg/ml in PBS followed by ultrapure water for 1 day each and lyophilizing). Error bars: standard error of the mean with n=11 to 16.

Fig. 16 shows that the macromolecule equilibrium

concentration in PEG gels is lower than in the supernatant solution, but macroporous gels enable the macromolecules to reach full concentration in the pores, which makes them eventually available to cells encapsulated in depth in the gels. A) A PEG gel formed from 4-arm-PEG-VS of 20 kg/mol at 1.5% (w/v) with PEG-dithiol 3.8 kg/mol cross-linker is immersed in a fluorescently tagged neutravidin solution and the concentration of neutravidin in the PEG gel compared to that in the supernatant and pores is measured from the respective fluorescence intensities. The concentration ratio is indicated under a fluorescence image showing the gel at the bottom and supernatant on top. B) Same for a fluorescently tagged succinylated chitosan. C) Same for a fluorescently tagged Dextran of 500 kg/mol.

Fig. 17 shows that macroporous PEG gels with tunable pore size can be formed by using a hyaluronan supplement to increase the viscosity and various amounts of dextran to increase the repulsion of PEG from the polysaccharide phase. Formation of macroporous PEG gels with pore size ranging from less than one micrometer to more than 50 micrometers is demonstrated in the figure (4-arm-PEG-vinylsulfone of 20 kg/mol at 1.5% (w/v) cross-linked with PEG-dithiol of 3.8 kg/mol in the presence of the polysaccharides indicated above the fluorescence micrographs. Afluorescent tag is bound to the PEG polymer for visualization).

Fig. 18 shows macroporous PEG gels formed with other cross-linking chemistries than the Michael addition of thiols on vinyl sulfones. Even though the pore size resulting from a particular polysaccharide concentration is different, the principle of the method still works the same. Transglutaminase cross-linked PEG is illustrated here by a PEG gel formed from a 4-arm-PEG precursor of 20 kg/mol end-functionalized with peptides which are substrates for the transglutaminase activated blood coagulation factor XIII (FKGG-ERCG and NQEQVSPL-ERCG).

Linear PEG-diacrylate of molecular weight 10 kg/mol was dissolved at 10% (w/v) in an aqueous buffer (glucose 100 mmol/L, TRIS 50 mmol/L, calcium 50 mmol/L). Dextran of molecular weight 450-550 kg/mol was dissolved at 5% (w/v) in the same buffer. The photoactivatable radical initiator Irgacure 2959 was dissolved at 0.2% (w/v) in the same buffer. The solutions were then combined with 40 $\mu$l of PEG solution, 40 $\mu$l of dextran solution, and 10 $\mu$l of Irgacure 2959 solution. The mix was a clear and homogeneous solution, that was placed between two coverslips with 1 mm spacing and exposed to UV at 365 nm with a light intensity of approximately 10 mW/cm^2 for 10 min for crosslinking. For assessment of pore formation, the resulting hydrogel was incubated for 20 min in a saturated solution of 4-methyl 7-thiocoumarin, which reacts with the few unreacted acrylate groups, thereby marking the PEG with blue fluorescence. The fluorescent porous PEG gel was imaged with two-photon excitated fluorescence with excitation at 710 nm and light collection between 400 and 500 nm, showing a 3D structured homogeneous porous structure.

In presence of activated factor XIII and calcium, the 4-arm-PEG precursor is cross-linked into a gel. Free radical polymerization of PEG-diacrylate is illustrated here with 10 kg/mol PEG-diacrylate (end-functionalized) and using 0.025% (w/v) of Irgacure 2959 as a radical initiator activated upon UV irradiation at 365 nm. Hyaluronan (HA) and dextran are used for the creation of macropores. Images are acquired with a microscope equipped with differential interference contrast (DIC) filters. Scale bars: 10 micrometers.

Fig. 19 shows a macroporous polyoxazoline hydrogel formed by Michael addition, fluorescently tagged and imaged with two-photon excited fluorescent laser-scanning confocal microscopy.

A 150 units long copolymer of 93% 2-ethyl 2-oxazoline and 7% of 2-carboxyethyl 2-oxazoline was used as starting material. This is a linear poly 2-ethyl 2-oxazoline that contains carboxylic acids useful to introduce cross-linkable moieties. A thiol containing derivative was obtained by standard EDC activation of the carboxylates for reaction with a two fold excess of 3,3'-dithiobis(propanoic dihydrazide) (DTPHY) in 300 mM MES buffer pH4.5, followed by reduction with a 10 fold excess of tricarboxyethyl phosphine (TCEP), followed by dialysis and lyophilization. A vinylsulfone derivative was obtained by reaction of the thiol derivative with 50 fold excess of divinyl sulfone in triethanolamine buffer, pH 8.0, under nitrogen atmosphere for 1h, followed by dialysis and lyophilization.

The thio- and vinylsulfone- derivatives of poly(2-ethyl 2-oxazoline) were resuspended at 10% (w/v) in mQ water and in 300mM triethanolamine buffer, pH8.0, respectively. Hyaluronan of molecular weight 1000 to 2000 kg/mol was dissolved at 7.5 mg/ml in the same buffer. The solutions were combined, with 20 $\mu$l of each polyoxazoline derivative for 40 $\mu$l of hyaluronan solution. For visualization of the porous structures, a small amount of thiol-conjugated fluorescent coumarin could be prereacted on the vinylsulfone functionalized polyoxazoline. After thorough mixing, gelation by Michael addition was left to proceed for 1h at room temperature, and the fluorescently tagged macroporous polyoxazoline hydrogel was imaged on a scanning confocal fluorescence microscope.

Fig. 20 shows the calcium spiking activity measured in rat cortical neurons encapsulated in PEG gels is due to glutamatergic excitation of the neurons and action potentials firing, as they get inhibited by the glutamatergic transmission inhibitors d,l-3[(±)-2-carboxypiperazin-4-yl]-propyl-1-phosphonic acid

(CPP) and 6-Cyano-7-nitroquinoxaline-2,3-dione (CNQX) and by the voltage-gated sodium channel inhibitor tetrodotoxin (TTX). Calcium spikes as reported by the fluorescent indicator Oregon Green BAPTA-1 are counted in the field of view of a 90 s fluorescence video acquired with a 5x objective on a widefield microscope.

## Claims

**1.** A method for providing a macroporous hydrogel, comprising the steps of

a) providing an aqueous solution comprising a first and a second solute, wherein

i) the first solute is a cross-linkable polymer selected from a cross-linkable derivative of a polyethylene glycol (PEG) and a cross-linkable derivative of a polyoxazoline (POx); and

ii) the second solute is a polysaccharide kosmotropic agent selected from the group comprising hyaluronan, mannuronan, carrageenan, heparin, chondroitin sulfate, dextran, hydroxypropyl dextran, pectin, alginate, gellan gum, cellulose, methylcellulose, ethylcellulose, cellulose acetate, starch and hydroxypropyl starch, wherein the concentration of said polysaccharide kosmotropic agent in said aqueous solution is 0.1 to 50% (w/v); and

b) adding to said mixture a cross-linking reagent able to cross-link said cross-linkable polymer, thus simultaneously initiating gelation of said cross-linkable polymer and phase-separation.

**2.** The method according to claim 1, wherein said cross-linkable polymer is selected from

a) the group comprising a linear or star-shaped polyethylene glycol, a polyethylene glycol diblock copolymer and a polyethylene glycol triblock copolymer,; or

b) the group comprising a polymethyloxazoline, a polyethyloxazoline, a polypropyloxazoline and a polybutyloxazoline; or

c) the group comprising a linear or star-shaped polyoxazoline and a copolymer of oxazoline,; or

d) the group comprising partially hydrolyzed poly 2-methyl oxazoline or poly 2-ethyl oxazoline

e) a polyethylene glycol diblock copolymer consisting of blocks of two monomers, one being polyethylene glycol, and the other one being selected from the group comprising polyethylene (PE), polylactide (PLA), polylactide-co-glycolide (PLGA), poly-ε-caprolactone (PCL) and polystyrene

f) a polyethylene glycol triblock copolymer consisting of blocks of two monomers, one being polyethylene glycol, the other one being selected from polypropylene glycol (PPG) and polylactide-co-glycolide (PLGA).

**3.** The method according to any one of the above claims, wherein said cross-linkable polymer comprises a reactive moiety selected from the group comprising acrylate, methacrylate, acrylamide, vinyl ester, maleimide, vinyl sulfone, alkyne, azide, aldehyde and thiol moieties.

**4.** The method according to any one of the above claims, wherein said cross-linkable polymer is vinylsulfone-modified and said cross-linking reagent is selected from the group comprising

a) a short linker molecule comprising two thiol (-SH) moieties; and

b) a star-shaped polyethylene glycol comprising primary thiol ($-CH_2SH$) moieties or a linear polyethylene glycol comprising primary thiol ($-CH_2SH$) moieties; and

c) a peptide containing two to one hundred amino acids, wherein two of said amino acids are cysteines; and

d) proteins containing two cysteines.

**5.** The method according to any one of the above claims, wherein the molecular weight of said cross-linkable polymer is 1,000 to 1,000,000 g/mol.

**6.** The method according to any one of the above claims, wherein the concentration of said cross-linkable polymer in said aqueous solution is 0.5 to 10% (w/v).

**7.** The method according to any one of the above claims, wherein said aqueous solution comprises 0.5 to 3% (w/v) of said cross-linkable derivative of a polyethylene glycol or 4 to 10% (w/v) of said cross-linkable derivative of a polyoxazoline.

**8.** The method according to any one of the above claims, wherein said polysaccharide kosmotropic agent is a polysaccharide selected from the group comprising pectin, alginate, gellan gum, cellulose, hyaluronan, mannuronan and dextran, and wherein said polysaccharide is **characterized by** a molecular weight in the range from 1,000 g/mol to 10,000,000 g/mol, particularly 10,000 g/mol to 2,000,000 g/mol, more particularly 100,000 g/mol to 1,000,000 g/mol, even more particularly approx. 150,000 g/mol.

**9.** The method according to any one of the above claims, wherein the concentration of said polysaccharide kosmotropic agent in said aqueous solution is 0.1 to 5% (w/v).

**10.** The method according to any one of claims 1 to 8, wherein said aqueous solution comprises 0.1 to 1% hyaluronan, or 0.5 to 5% mannuronan, or 0.1 to 20% dextran, or a mixture thereof.

**11.** The method according to any one of the above claims, wherein said cross-linkable polymer is 20 kDa 4-arm-PEG-thiol modified with vinylsulfone-moieties, said polysaccharide is mannuronan, and said cross-linking reagent is a matrix metalloproteinase cleavable peptide.

**12.** The method according to any one of the above claims, wherein said aqueous solution is **characterized by** a pH of 7.0-8.0 and addition of said cross-linking agent occurs at 20°C to 40°C.

**13.** The method according to any one of the above claims, wherein a cell is added to said aqueous solution prior to cross-linking.

**14.** A macroporous hydrogel obtained or obtainable by the method of any one of claims 1 to 13.

**Patentansprüche**

**1.** Ein Verfahren zur Bereitstellung eines makroporösen Hydrogels, umfassend die Schritte

a) Bereitstellen einer wässrigen Lösung, umfassend einer ersten und einer zweiten gelösten Substanz, wobei

i.) die erste gelöste Substanz ein vernetzbares Polymer ist, ausgewählt aus einem vernetzbaren Derivat eines Polyethylenglykols (PEG) und einem vernetzbaren Derivat eines Polyoxazolins (POx); und
ii.) die zweite gelöste Substanz ein kosmotropes Polysaccharidmittel ist, ausgewählt aus der Gruppe umfassend Hyaluronan, Mannuronan, Carrageenan, Heparin, Chondroitinsulfat, Dextran, Hydroxypropyldextran, Pectin, Alginat, Gellangummi, Cellulose, Methylcellulose, Ethylcellulose, Celluloseacetat, Stärke und Hydroxypropylstärke,

wobei die Konzentration des kosmotropen Polysaccharidmittels in der wässrigen Lösung 0,1 bis 50% (w/v) beträgt; und
b) Zugabe eines Vernetzungsreagens zu der Mischung, das in der Lage ist, das vernetzbare Polymer zu vernetzen, wodurch gleichzeitig die Gelierung des vernetzbaren Polymers und die Phasentrennung eingeleitet werden.

**2.** Das Verfahren nach Anspruch 1, wobei das vernetzbare Polymer ausgewählt ist aus

a) der Gruppe, umfassend ein lineares oder sternförmiges Polyethylenglykol, ein Polyethylenglykol-Diblock-Copolymer und ein Polyethylenglykol-Triblock-Copolymer, oder
b) der Gruppe, umfassend ein Polymethyloxazolin, ein Polyethyloxazolin, ein Polypropyloxazolin und ein Polybutyloxazolin; oder
c) der Gruppe, umfassend ein lineares oder sternförmiges Polyoxazolin und ein Copolymer von Oxazolin; oder
d) der Gruppe, umfassend teilweise hydrolysiertes Poly-2-methyloxazolin oder Poly-2-ethyloxazolin;
e) einem Polyethylenglykol-Diblock-Copolymer, bestehend aus Blöcken aus zwei Monomeren, von denen eines Polyethylenglykol ist und das andere ausgewählt ist aus der Gruppe umfassend Polyethylen (PE), Polylactid (PLA), Polylactid-co-glycolid (PLGA), Poly-ε-caprolacton (PCL) und Polystyrol;
f) einem Polyethylenglykol-Triblock-Copolymer, bestehend aus Blöcken von zwei Monomeren, wobei eines Polyethylenglykol ist und das andere ausgewählt ist aus Polypropylenglykol (PPG) und Polylactid-co-glycolid (PLGA).

**3.** Das Verfahren nach einem der vorherigen Ansprüche, wobei das vernetzbare Polymer eine reaktive Einheit umfasst, die aus der Gruppe ausgewählt ist umfassend Acrylat-, Methacrylat-, Acrylamid-, Vinylester-, Maleimid-, Vinylsulfon-, Alkin-, Azid-, Aldehyd- und Thiol-Einheiten.

**4.** Das Verfahren nach einem der vorherigen Ansprüche, wobei das vernetzbare Polymer Vinylsulfon-modifiziert ist und das Vernetzungsreagenz ausgewählt ist aus der Gruppe, die umfasst

a) ein kurzes Linkermolekül, das zwei Thiol-(-SH)-Anteile umfasst; und
b) ein sternförmiges Polyethylenglykol, umfassend primäre Thiol ($-CH_2SH$)-Einheiten, oder ein lineares Polyethylenglykol, umfassend primäre Thiol ($-CH_2SH$)-Einheiten; und
c) ein Peptid, das zwei bis einhundert Aminosäuren enthält, wobei zwei der Aminosäuren Cysteine sind; und
d) Proteine, die zwei Cysteine enthalten.

**5.** Das Verfahren nach einem der vorherigen Ansprüche, wobei das Molekulargewicht des vernetzbaren Polymers 1.000 bis 1.000.000 g/mol beträgt.

6. Das Verfahren nach einem der vorherigen Ansprüche, wobei die Konzentration des vernetzbaren Polymers in der wässrigen Lösung 0,5 bis 10 % (w/v) beträgt.

7. Das Verfahren nach einem der vorherigen Ansprüche, wobei die wässrige Lösung 0,5 bis 3 % (w/v) des vernetzbaren Derivats eines Polyethylenglykols oder 4 bis 10 % (w/v) des vernetzbaren Derivats eines Polyoxazolins enthält.

8. Das Verfahren nach einem der vorherigen Ansprüche, wobei das kosmotrope Polysaccharidmittel ein Polysaccharid ist, das aus der Gruppe ausgewählt ist umfassend Pektin, Alginat, Gellangummi, Cellulose, Hyaluronan, Mannuronan und Dextran, und wobei das Polysaccharid durch ein Molekulargewicht gekennzeichnet ist im Bereich von 1. 000 g/mol bis 10.000.000 g/mol, insbesondere 10.000 g/mol bis 2.000.000 g/mol, weiter insbesondere 100.000 g/mol bis 1.000.000 g/mol, noch weiter insbesondere ca. 150.000 g/mol.

9. Das Verfahren nach einem der vorherigen Ansprüche, wobei die Konzentration des kosmotropen Polysaccharidmittels in der wässrigen Lösung 0,1 bis 5% (w/v) beträgt.

10. Das Verfahren nach einem der Ansprüche 1 bis 8, wobei die wässrige Lösung 0,1 bis 1 % Hyaluronan oder 0,5 bis 5 % Mannuronan oder 0,1 bis 20 % Dextran oder eine Mischung davon enthält.

11. Das Verfahren nach einem der vorherigen Ansprüche, wobei das vernetzbare Polymer ein 20 kDa 4-armiges PEG-Thiol ist, das mit Vinylsulfon-Molekülen modifiziert ist, das Polysaccharid Mannuronan ist und das Vernetzungsreagenz ein durch Matrixmetalloproteinase spaltbares Peptid ist.

12. Das Verfahren nach einem der vorherigen Ansprüche, wobei die wässrige Lösung durch einen pH-Wert von 7,0-8,0 gekennzeichnet ist und die Zugabe des Vernetzungsreagenz bei 20°C bis 40°C erfolgt.

13. Das Verfahren nach einem der vorherigen Ansprüche, wobei der wässrigen Lösung vor dem Vernetzen eine Zelle zugesetzt wird.

14. Ein makroporöses Hydrogel, erhalten oder erhältlich durch das Verfahren nach einem der Ansprüche 1 bis 13.

**Revendications**

1. Procédé de fourniture d'un hydrogel macroporeux, comprenant les étapes consistant à

a) fournir une solution aqueuse comprenant un premier et un second soluté, dans lequel

i) le premier soluté est un polymère réticulable sélectionné parmi un dérivé réticulable d'un polyéthylène glycol (PEG) et un dérivé réticulable d'une polyoxazoline (POx) ; et
ii) le second soluté est un agent kosmotropique de polysaccharide sélectionné parmi le groupe comprenant l'hyaluronane, le mannuronane, le carraghénane, l'héparine, le chondroïtine sulfate, le dextrane, le dextrane hydroxypropylé, la pectine, l'alginate, la gomme gellane, la cellulose, la méthylcellulose, l'éthylcellulose, l'acétate de cellulose, l'amidon et l'amidon hydroxypropylé,

dans lequel la concentration en ledit agent kosmotropique de polysaccharide dans ladite solution aqueuse est de 0,1 à 50 % (p/v) ; et
b) ajouter audit mélange un réactif de réticulation capable de réticuler ledit polymère réticulable, initiant ainsi simultanément la gélification dudit polymère réticulable et la séparation de phases.

2. Procédé selon la revendication 1, dans lequel ledit polymère réticulable est sélectionné parmi

a) le groupe comprenant un polyéthylène glycol linéaire ou en forme d'étoile, un copolymère dibloc de polyéthylène glycol et un copolymère tribloc de polyéthylène glycol ; ou
b) le groupe comprenant une polyméthyloxazoline, une polyéthyloxazoline, une polypropyloxazoline et une polybutyloxazoline ; ou
c) le groupe comprenant une polyoxazoline linéaire ou en forme d'étoile et un copolymère d'oxazoline ; ou
d) le groupe comprenant de la poly 2-méthyl oxazoline ou poly 2-éthyl oxazoline partiellement hydrolysée ;
e) un copolymère dibloc de polyéthylène glycol constitué de blocs de deux monomères, un étant du polyéthylène glycol, et l'autre étant sélectionné parmi le groupe comprenant le polyéthylène (PE), l'acide polylactique (PLA), l'acide polylactique-co-glycolique (PLGA), la poly-ε-caprolactone (PCL) et le polystyrène ;
f) un copolymère tribloc de polyéthylène glycol constitué de blocs de deux monomères, un étant du polyéthylène glycol, l'autre étant sélectionné

parmi du polypropylène glycol (PPG) et de l'acide polylactique-co-glycolique (PLGA).

**3.** Procédé selon l'une quelconque des revendications précédentes, dans lequel ledit polymère réticulable comprend un groupe caractéristique réactif sélectionné parmi le groupe comprenant des groupes caractéristiques d'acrylate, de méthacrylate, d'acrylamide, d'ester de vinyle, de maléimide, de vinylsulfone, d'alkyne, d'azide, d'aldéhyde et de thiol.

**4.** Procédé selon l'une quelconque des revendications précédentes, dans lequel ledit polymère réticulable est modifié par une vinylsulfone et ledit réactif de réticulation est sélectionné parmi le groupe comprenant

a) une molécule de coupleur courte comprenant deux groupes caractéristiques de thiol (-SH) ; et
b) un polyéthylène glycol en forme d'étoile comprenant des groupes caractéristiques de thiol primaire ($-CH_2SH$) ou un polyéthylène glycol linéaire comprenant des groupes caractéristiques de thiol primaire ($-CH_2SH$) ; et
c) un peptide contenant deux à cent acides aminés, dans lequel deux desdits acides aminés sont des cystéines ; et
d) des protéines contenant deux cystéines.

**5.** Procédé selon l'une quelconque des revendications précédentes, dans lequel le poids moléculaire dudit polymère réticulable est de 1000 à 1 000 000 g/mol.

**6.** Procédé selon l'une quelconque des revendications précédentes, dans lequel la concentration en ledit polymère réticulable dans ladite solution aqueuse est de 0,5 à 10 % (p/v).

**7.** Procédé selon l'une quelconque des revendications précédentes, dans lequel ladite solution aqueuse comprend 0,5 à 3 % (p/v) dudit dérivé réticulable d'un polyéthylène glycol ou 4 to 10 % (p/v) dudit dérivé réticulable d'une polyoxazoline.

**8.** Procédé selon l'une quelconque des revendications précédentes, dans lequel ledit agent kosmotropique de polysaccharide est un polysaccharide sélectionné parmi le groupe comprenant de la pectine, de l'alginate, de la gomme gellane, de la cellulose, de l'hyaluronane, du mannuronane et du dextrane, et dans lequel ledit polysaccharide est **caractérisé par** un poids moléculaire dans la plage de 1000 g/mol à 10 000 000 g/mol, notamment 10 000 g/mol à 2 000 000 g/mol, plus particulièrement 100 000 g/mol à 1 000 000 g/mol, encore plus particulièrement d'approx. 150 000 g/mol.

**9.** Procédé selon l'une quelconque des revendications précédentes, dans lequel la concentration en ledit agent kosmotropique de polysaccharide dans ladite solution aqueuse est de 0,1 à 5 % (p/v).

**10.** Procédé selon l'une quelconque des revendications 1 à 8, dans lequel ladite solution aqueuse comprend 0,1 à 1 % de hyaluronane, ou 0,5 à 5 % de mannuronane, ou 0,1 à 20 % de dextrane, ou un mélange de ceux-ci.

**11.** Procédé selon l'une quelconque des revendications précédentes, dans lequel ledit polymère réticulable est un PEG-thiol à 4 bras de 20 kDa modifié avec des groupes caractéristiques de vinylsulfone, ledit polysaccharide est le mannuronane, et ledit réactif de réticulation est un peptide clivable par une métalloprotéinase de la matrice.

**12.** Procédé selon l'une quelconque des revendications précédentes, dans lequel ladite solution aqueuse est **caractérisée par** un pH de 7,0 à 8,0, et l'addition dudit agent de réticulation se produit à une température de 20°C à 40°C.

**13.** Procédé selon l'une quelconque des revendications précédentes, dans lequel une cellule est ajoutée à ladite solution aqueuse avant la réticulation.

**14.** Hydrogel macroporeux obtenu ou pouvant être obtenu par le procédé selon l'une quelconque des revendications 1 à 13.

Fig. 1

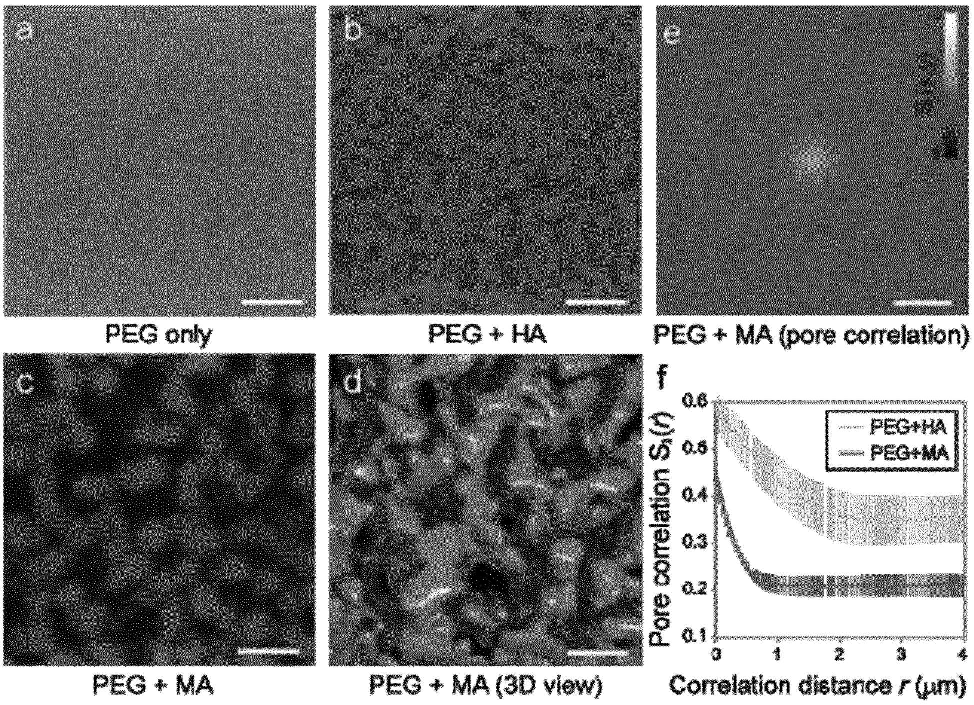
a
b
e
PEG only
PEG + HA
PEG + MA (pore correlation)
c
d
f
PEG + MA
PEG + MA (3D view)
PEG+HA
PEG+MA
Pore correlation $S_I(r)$
Correlation distance $r$ (μm)

Fig. 2

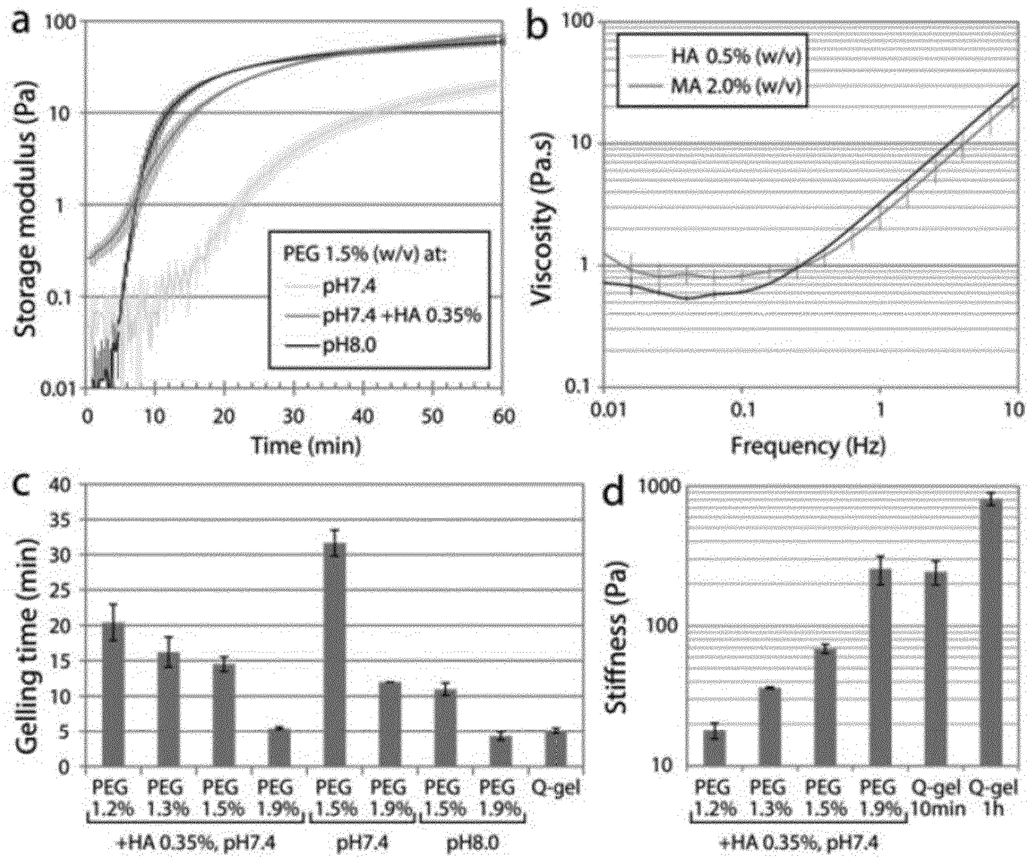
a
Storage modulus (Pa)
Time (min)
PEG 1.5% (w/v) at:
pH7.4
pH7.4 +HA 0.35%
pH8.0
b
HA 0.5% (w/v)
MA 2.0% (w/v)
Viscosity (Pa.s)
Frequency (Hz)
c
Gelling time (min)
PEG 1.2% PEG 1.3% PEG 1.5% PEG 1.9% PEG 1.5% PEG 1.9% PEG 1.5% PEG 1.9% Q-gel
+HA 0.35%, pH7.4
pH7.4
pH8.0
d
Stiffness (Pa)
PEG 1.2% PEG 1.3% PEG 1.5% PEG 1.9% Q-gel 10min Q-gel 1h
+HA 0.35%, pH7.4

Fig. 3

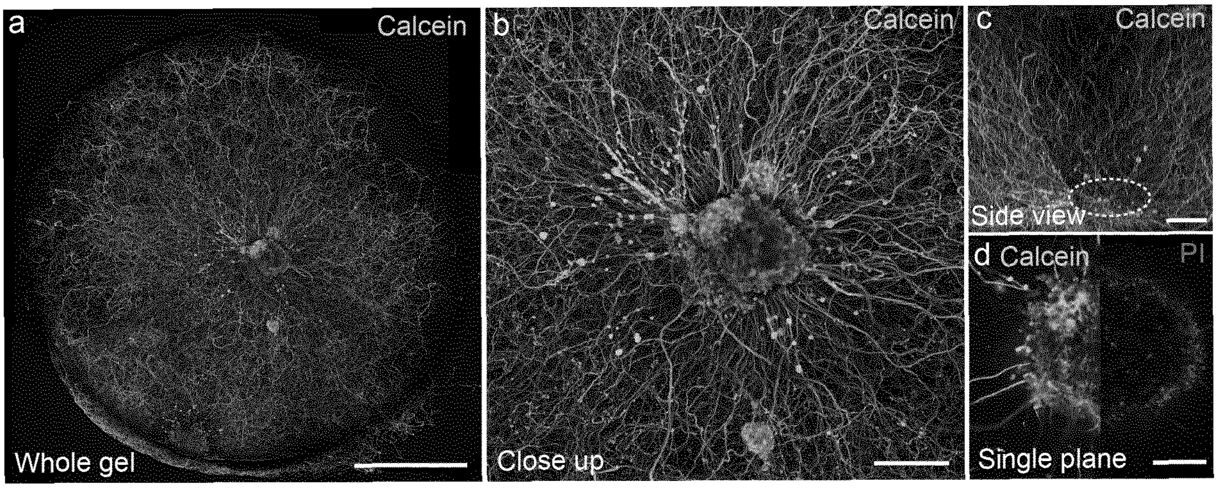


Fig. 4

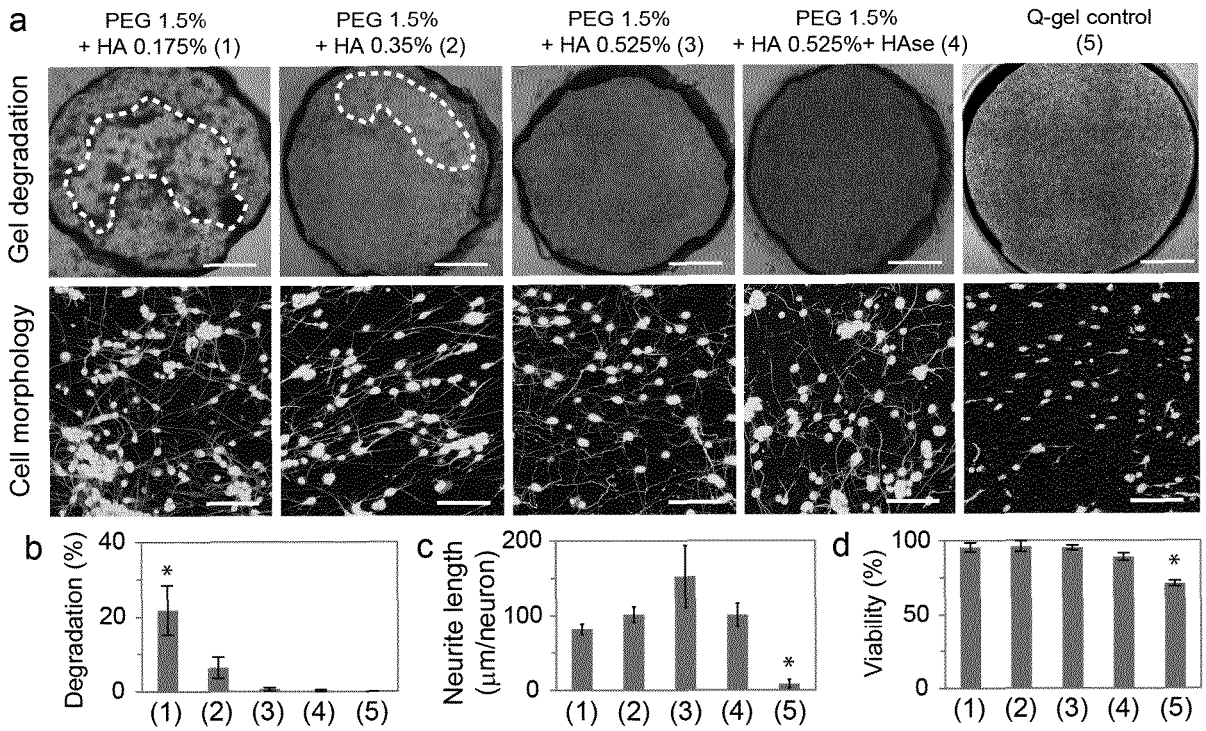


Fig. 5

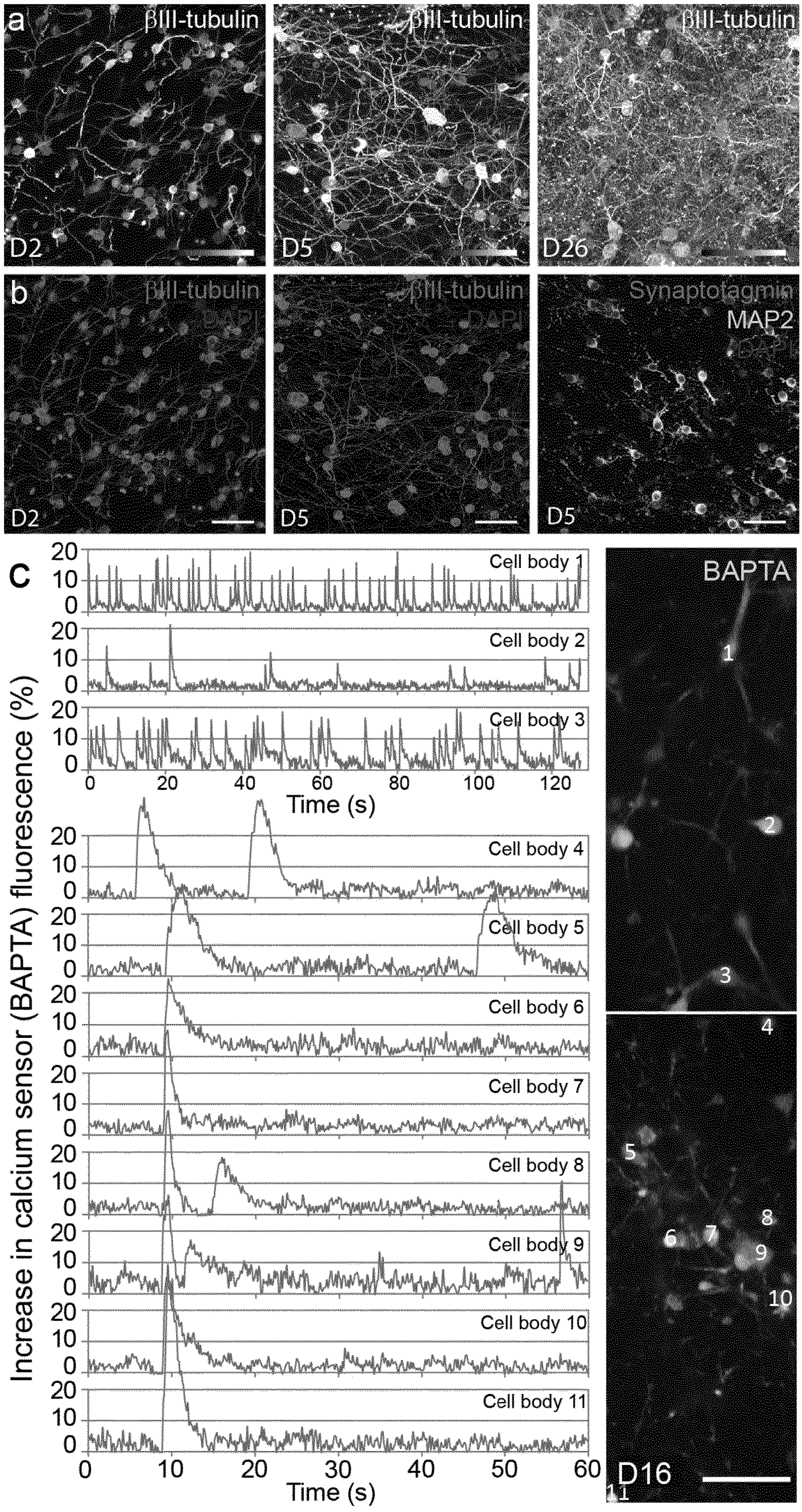
a
βIII-tubulin
D2
D5
D26
b
βIII-tubulin
DAPI
Synaptotagmin
MAP2
DAPI
c
Cell body 1
Cell body 2
Cell body 3
Cell body 4
Cell body 5
Cell body 6
Cell body 7
Cell body 8
Cell body 9
Cell body 10
Cell body 11
Increase in calcium sensor (BAPTA) fluorescence (%)
Time (s)
BAPTA
D16

Fig. 6

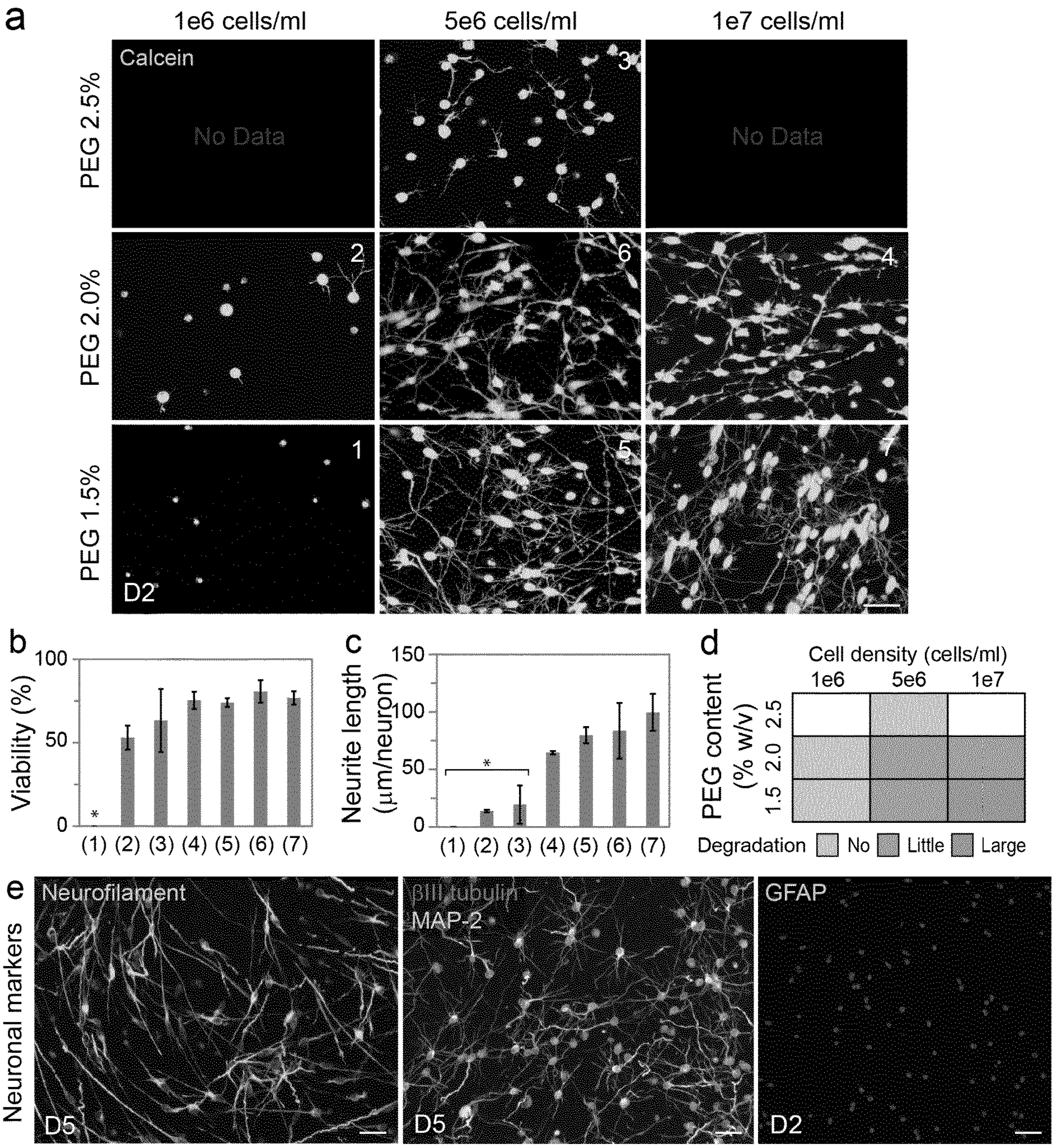
a
1e6 cells/ml
5e6 cells/ml
1e7 cells/ml
PEG 2.5%
PEG 2.0%
PEG 1.5%
Calcein
No Data
No Data
D2
b
Viability (%)
(1) (2) (3) (4) (5) (6) (7)
c
Neurite length (µm/neuron)
(1) (2) (3) (4) (5) (6) (7)
d
Cell density (cells/ml)
1e6 5e6 1e7
PEG content (% w/v)
2.5 2.0 1.5
Degradation No Little Large
e
Neuronal markers
Neurofilament
βIII tubulin
MAP-2
GFAP
D5
D5
D2

Fig 7

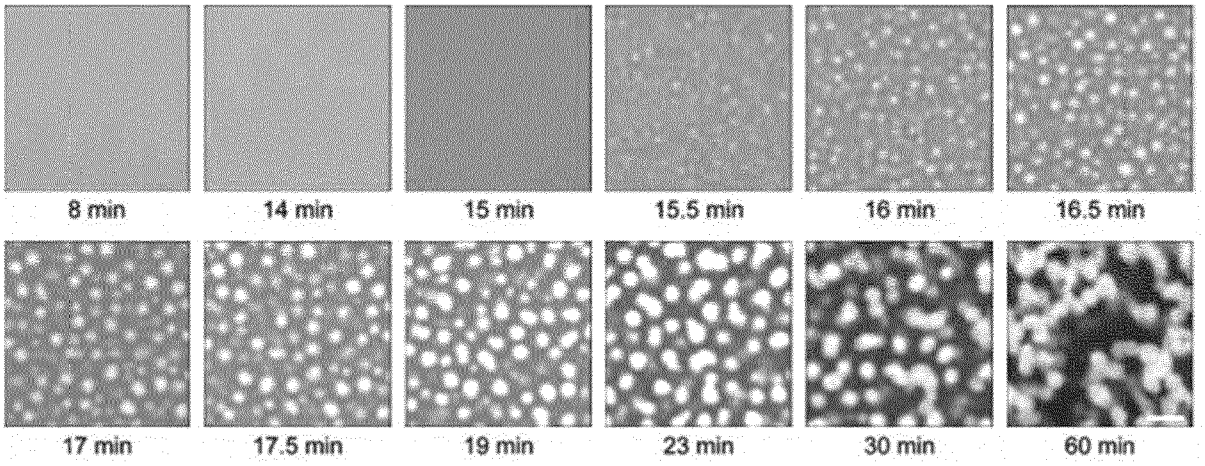
8 min
14 min
15 min
15.5 min
16 min
16.5 min
17 min
17.5 min
19 min
23 min
30 min
60 min

Fig. 8

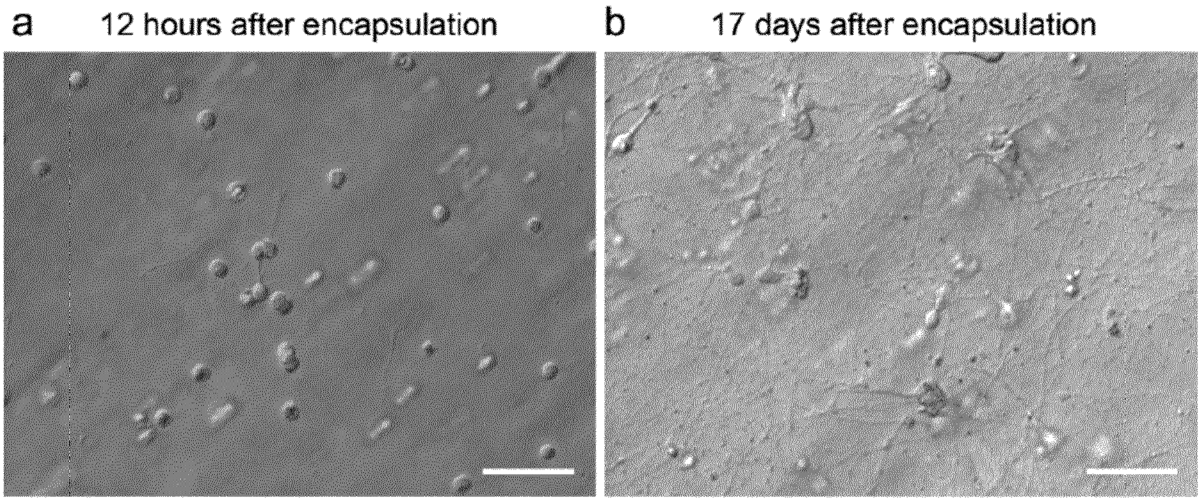
a
12 hours after encapsulation
b
17 days after encapsulation

Fig. 9

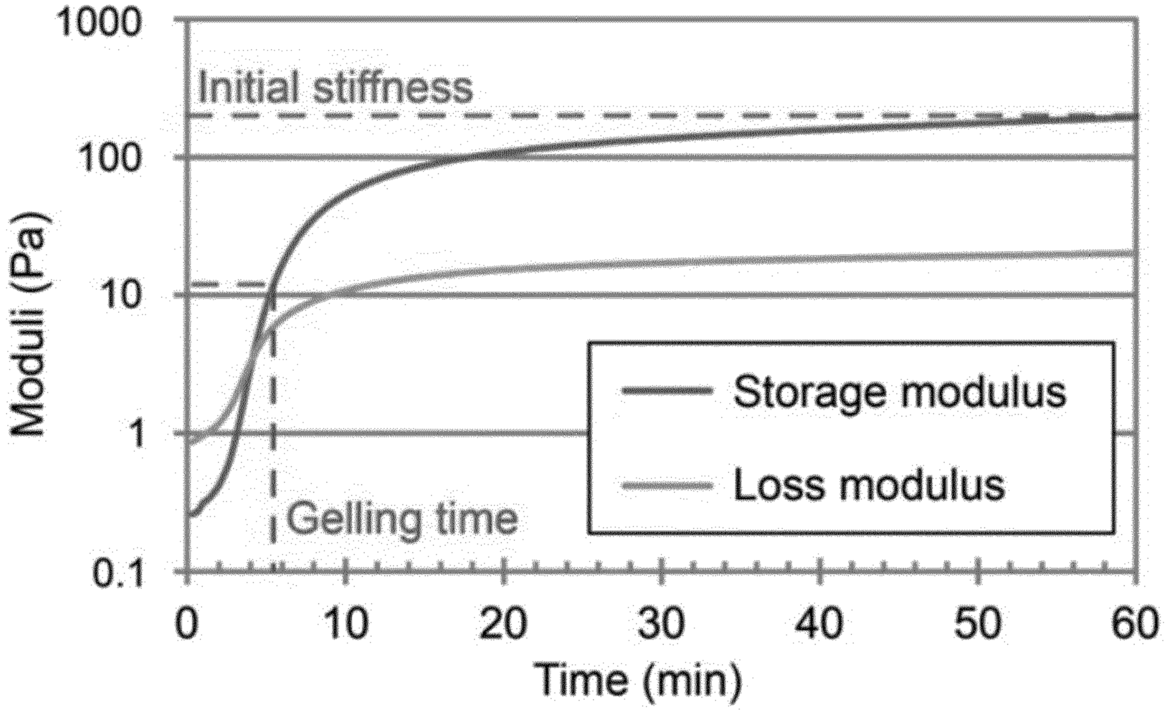
1000
100
10
1
0.1
Moduli (Pa)
Initial stiffness
Gelling time
Storage modulus
Loss modulus
0
10
20
30
40
50
60
Time (min)

Fig. 10

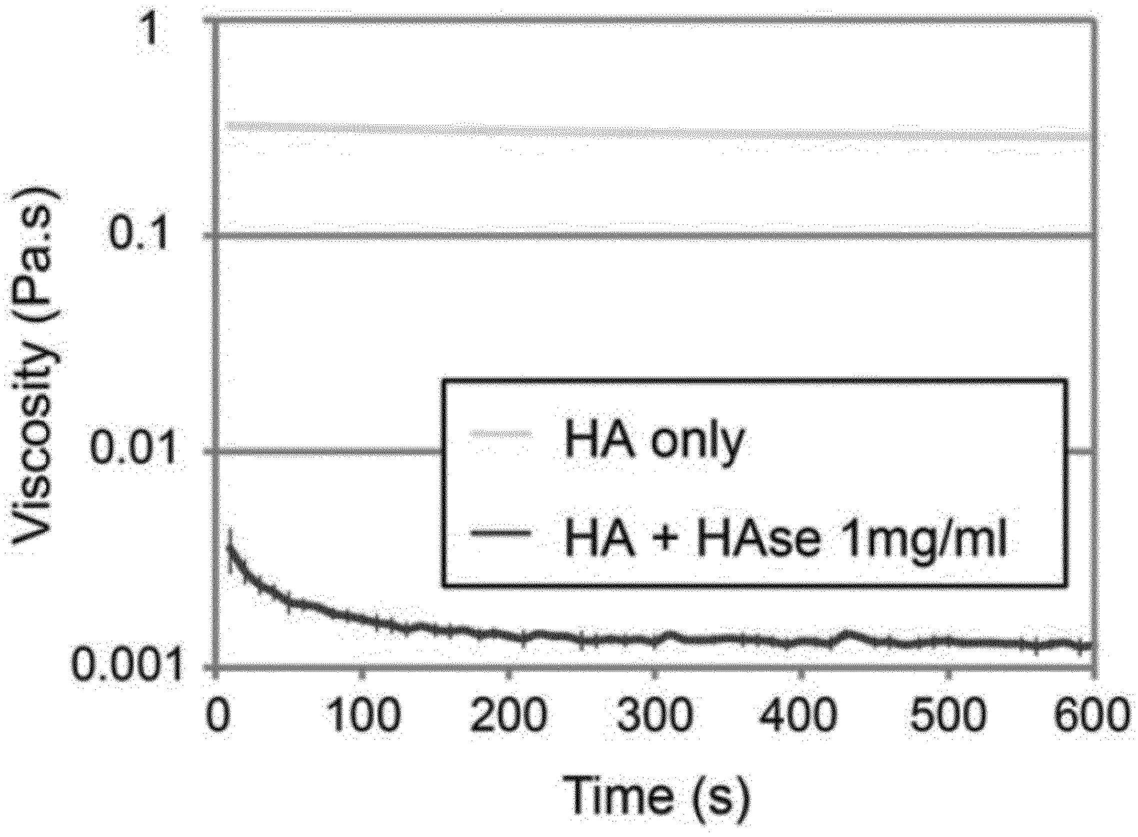
1
0.1
0.01
0.001
Viscosity (Pa.s)
HA only
HA + HAse 1mg/ml
0
100
200
300
400
500
600
Time (s)

Fig. 11

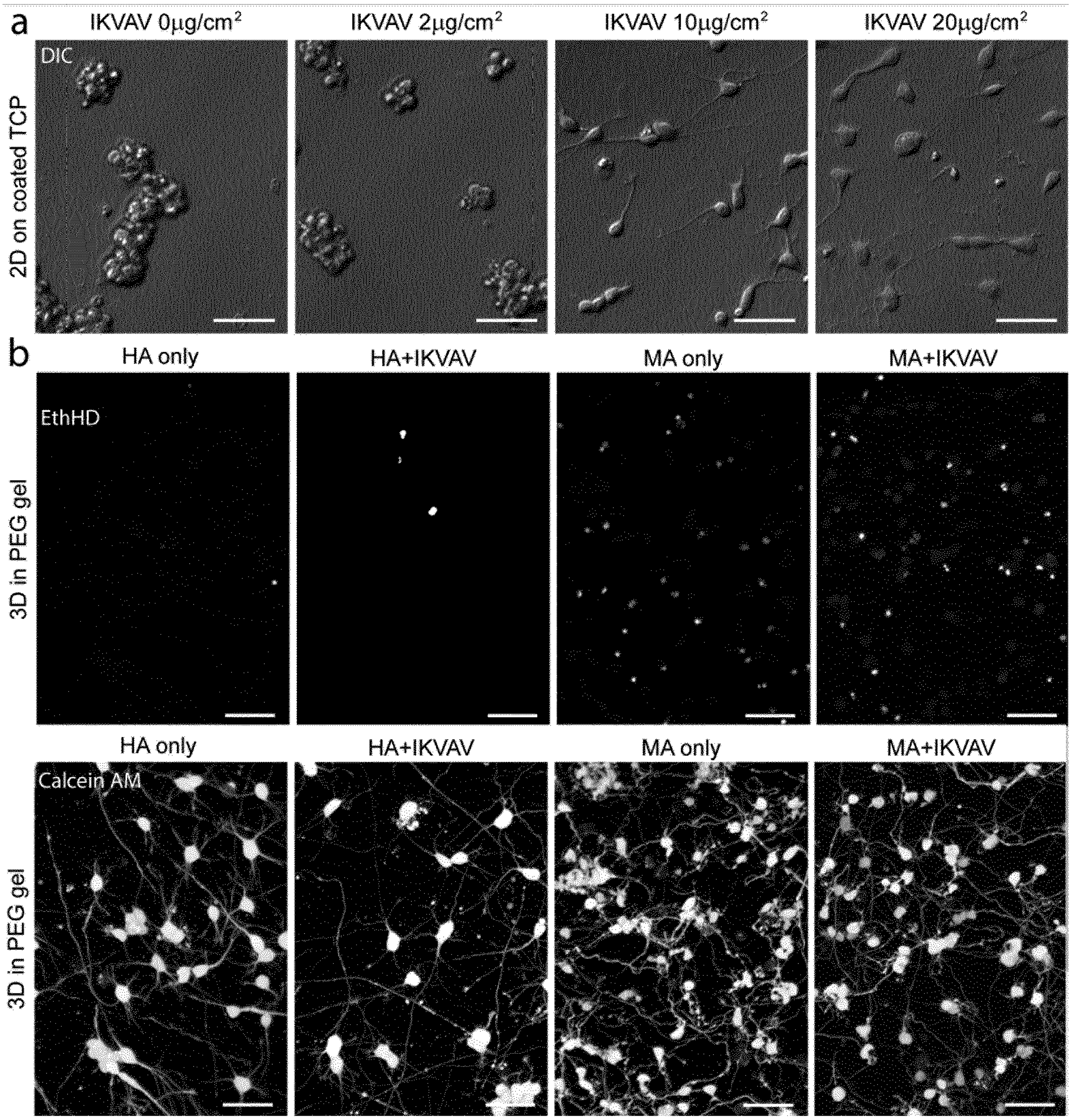
a
IKVAV 0μg/cm2
IKVAV 2μg/cm2
IKVAV 10μg/cm2
IKVAV 20μg/cm2
DIC
2D on coated TCP
b
HA only
HA+IKVAV
MA only
MA+IKVAV
EthHD
3D in PEG gel
HA only
HA+IKVAV
MA only
MA+IKVAV
Calcein AM
3D in PEG gel

Fig. 12

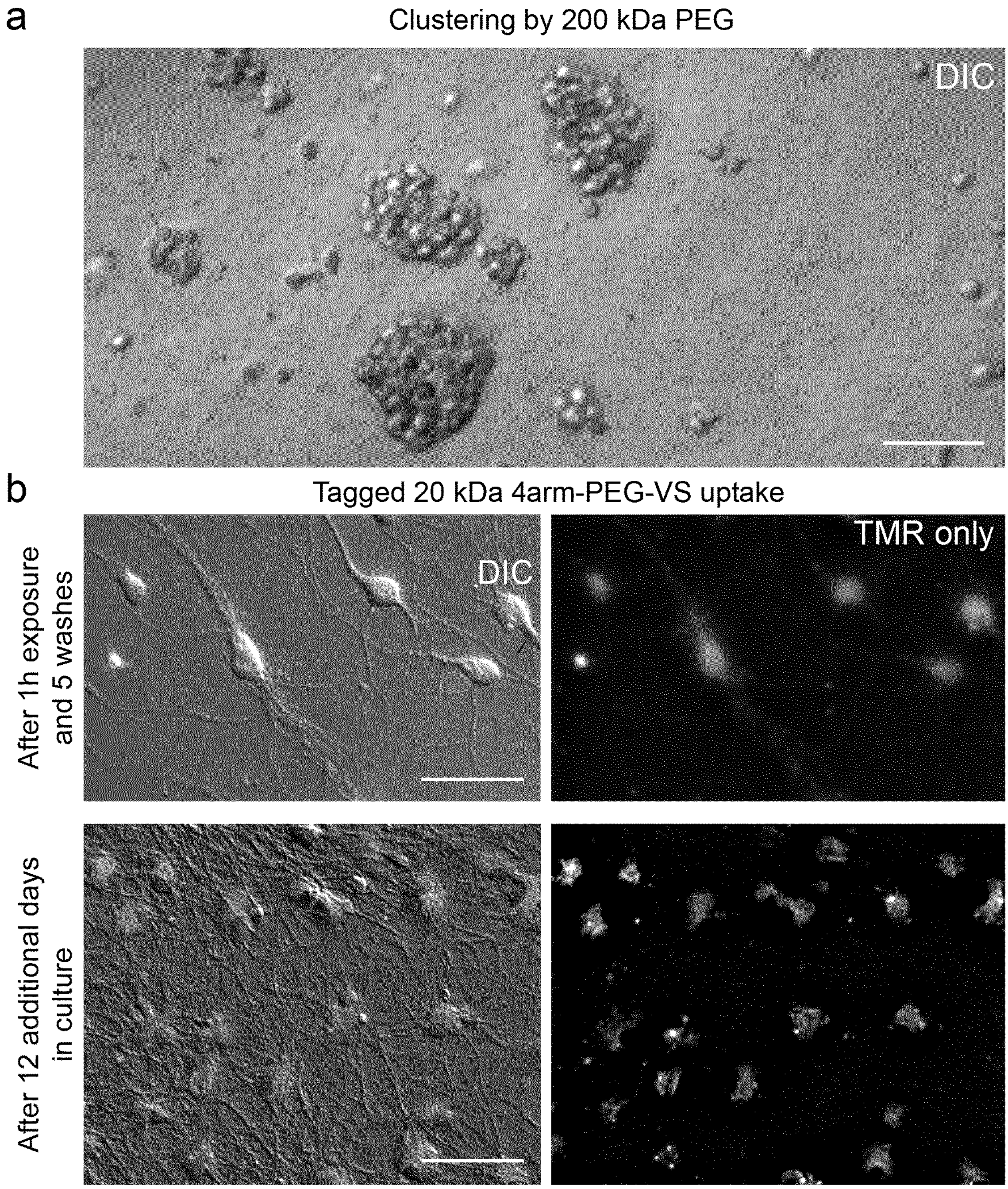
a
Clustering by 200 kDa PEG
DIC
b
Tagged 20 kDa 4arm-PEG-VS uptake
DIC
TMR only
After 1h exposure and 5 washes
After 12 additional days in culture

Fig. 13

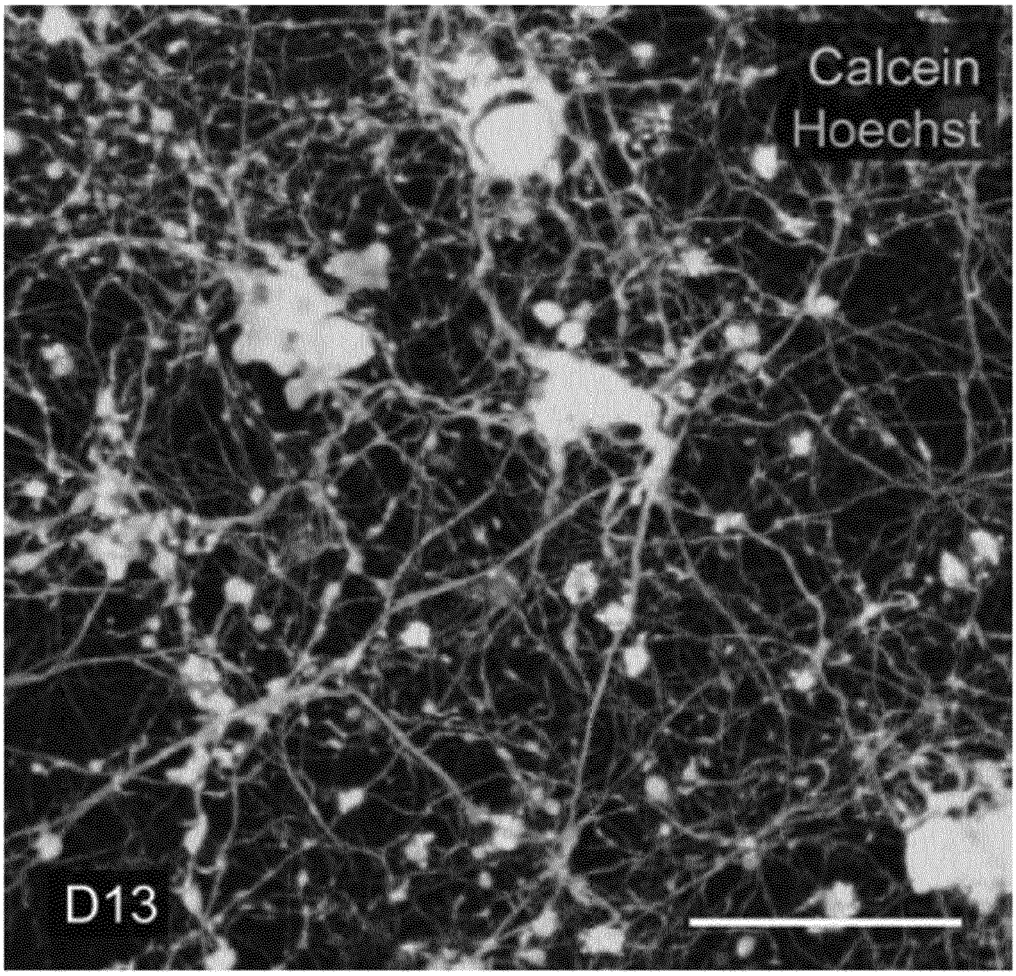


Fig. 14

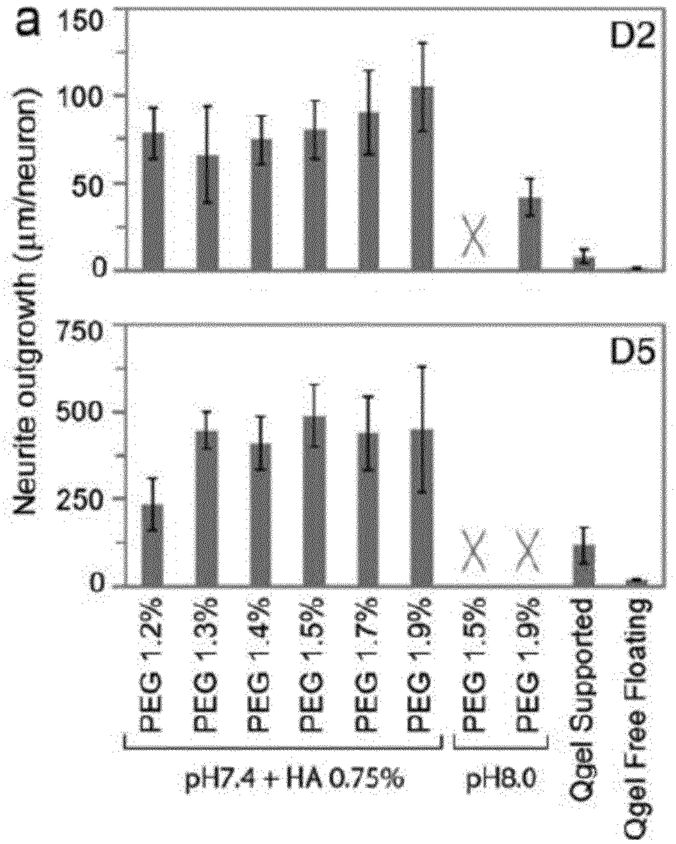
a
D2
D5
Neurite outgrowth (µm/neuron)
150
100
50
0
750
500
250
0
PEG 1.2%
PEG 1.3%
PEG 1.4%
PEG 1.5%
PEG 1.7%
PEG 1.9%
PEG 1.5%
PEG 1.9%
Qgel Supported
Qgel Free Floating
pH7.4 + HA 0.75%
pH8.0

Fig. 15

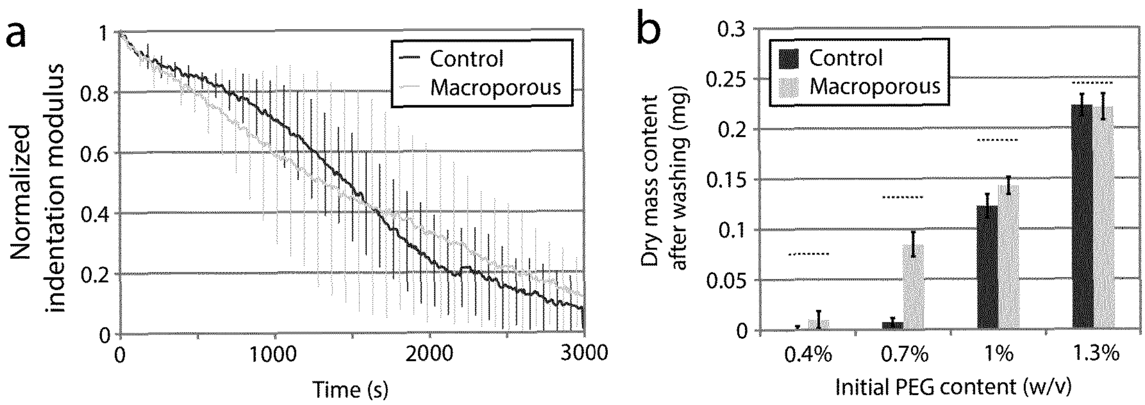


Fig. 16

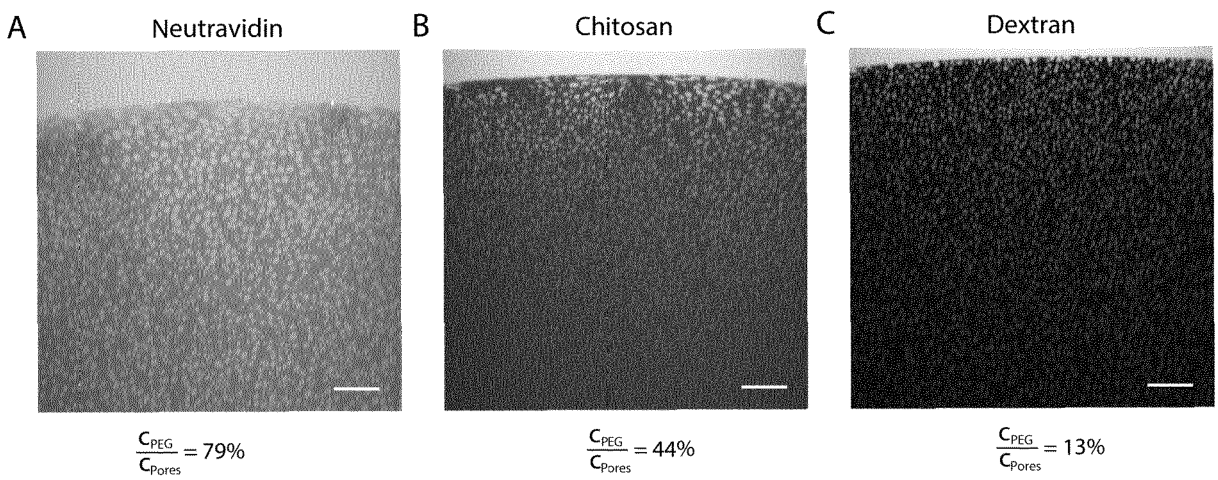


Fig. 17

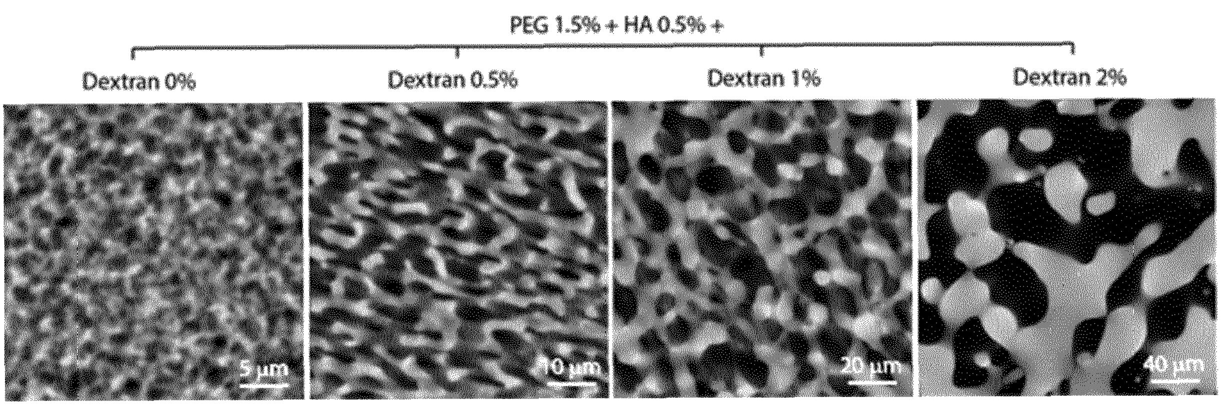

Fig. 18

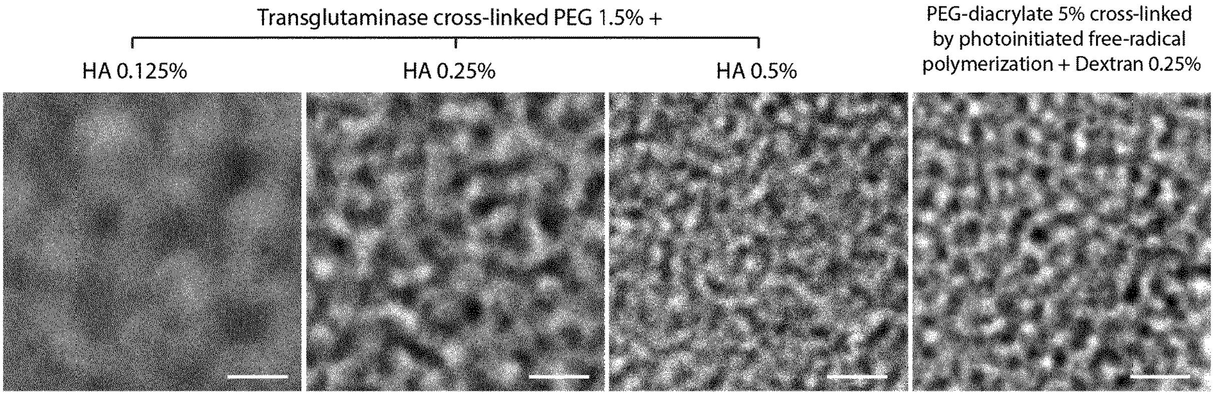
Transglutaminase cross-linked PEG 1.5% +
HA 0.125%
HA 0.25%
HA 0.5%
PEG-diacrylate 5% cross-linked by photoinitiated free-radical polymerization + Dextran 0.25%

Fig. 19

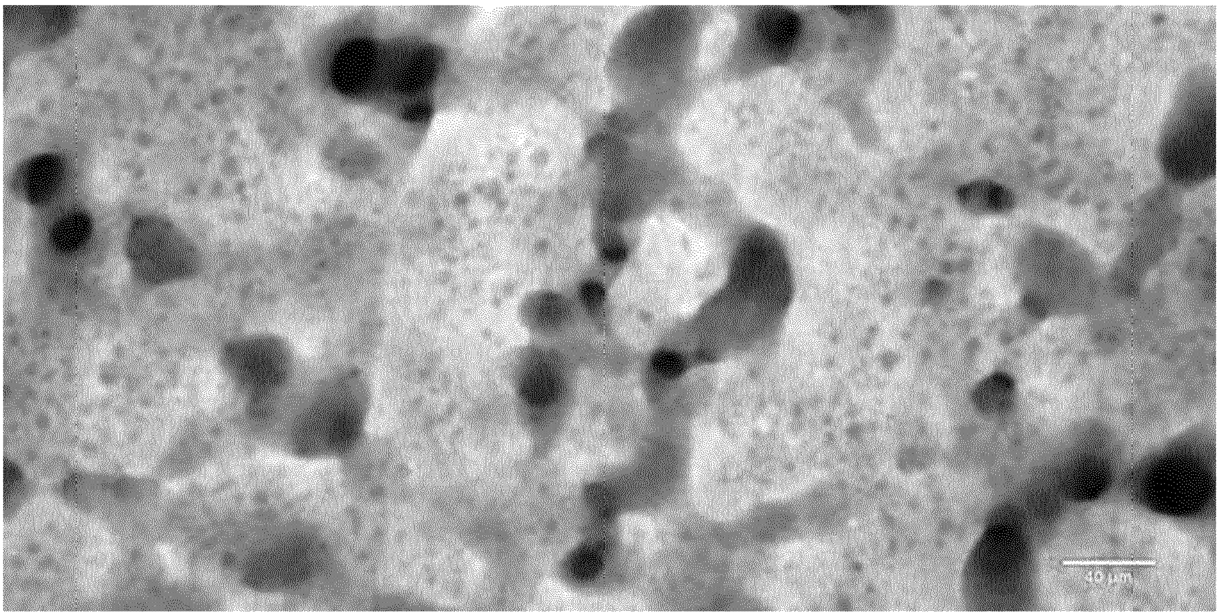

Fig. 20

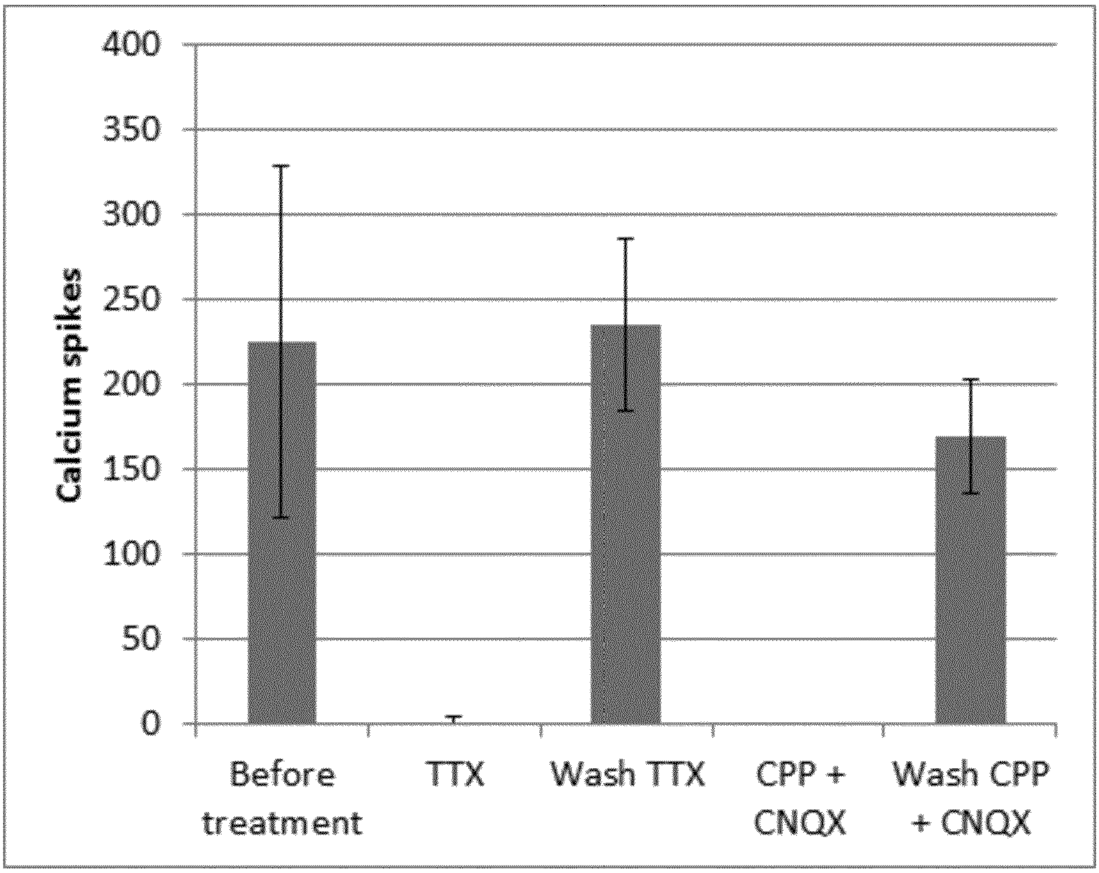

400
350
300
250
200
150
100
50
0
Calcium spikes
Before treatment
TTX
Wash TTX
CPP + CNQX
Wash CPP + CNQX

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014343324 A


### Non-patent literature cited in the description


- *CHEMICAL ABSTRACTS,* 25322-68-3
- *CHEMICAL ABSTRACTS,* 115-77-5
- *CHEMICAL ABSTRACTS,* 4704-94-3
- *CHEMICAL ABSTRACTS,* 126-58-9
- *CHEMICAL ABSTRACTS,* 78-24-0
- *CHEMICAL ABSTRACTS,* 30432-16-7
- *CHEMICAL ABSTRACTS,* 9004-61-9
- *CHEMICAL ABSTRACTS,* 6814-36-4
- **ANDRE STRIEGEL ; WALLACE W. YAU ; JOSEPH J. KIRKLAND ; DONALD D. BLY.** Modern Size-Exclusion Liquid Chromatography: Practice of Gel Permeation and Gel Filtration Chromatography. John Wiley & Sons, 2009
- **NAGASE, H.** *Biopolymers,* 1996, vol. 40, 339-416
- **LUTOLF, B. M. P.** *Adv. Mater.,* 2003, vol. 15, 888-892
- **ELLMAN, G. L.** *Arch. Biochem. Biophys.,* 1959, 70-77
- **LUTOLF, M.P.** *Biomacromolecules,* 2003, vol. 4, 713-722
- **YAO, W.** *Environ. Sci. Technol.,* 2001, vol. 35, 1197-201
- **ERTESVAG, H.** *Methods Biotechnol.,* 1999, vol. 10, 71-78
- **TASHIRO, K.** *J. Biol. Chem.,* 1989, vol. 264, 16174-82
- **BERRYMAN, J. G.** *J. Appl. Phys.,* 1985, vol. 57, 2374-2384
- **BUERLI, T.** *Nat. Protoc.,* 2007, vol. 2, 3090-101
- **LONGAIR, M. H.** *Bioinformatics,* 2011, vol. 27, 2453-2454
- **DEHMELT, L.** *BMC Neurosci.,* 2011, vol. 12, 100